# EUROPÄISCHE PATENTANMELDUNG

(11) **EP 2 581 863 A1**
(43) Veröffentlichungstag der Anmeldung: **17.04.2013**
(21) Anmeldenummer: 12188356.5
(22) Anmeldetag: 12.10.2012
(51) Int. Cl.: G06Q 10/08, G06Q 50/22, G06F 19/00

(54) **Vorrichtung und Verfahren zum Zusammenstellen von Instrumentensets**

(30) Priorität: 13.10.2011 DE 102011054452
(71) Anmelder: How to Organize (H2O) GmbH, 10715 Berlin (DE)
(72) Erfinder: Gloger, Oliver, 10967 Berlin (DE); Abri, Omid, 10129 Berlin (DE)
(74) Vertreter: Witte, Weller & Partner

(57) **Zusammenfassung**

Die Erfindung betrifft eine Vorrichtung zum Zusammenstellen von Instrumentensets, mit einer Datenverarbeitungsanlage (14) und einer Feinerkennungseinheit (12), wobei die Datenverarbeitungsanlage (14) eine Anzeigeeinheit (38), eine Datenbank, eine erste Schnittstelle und eine Auswerteeinheit aufweist, und wobei die Feinerkennungseinheit (12) eine erste Auflage (16) zum Auflegen der Instrumente (58, 64) eines Instrumentensets und eine erste Kamera (18) aufweist, und die erste Kamera (18) so angeordnet und ausgerichtet ist, dass sie Bilddaten von zu erkennenden Instrumenten (58, 64) auf der ersten Auflage (16) aus zumindest einer Perspektive erfassen kann, und die Datenverarbeitungsanlage (14) so ausgestaltet ist, dass sie über die erste Schnittstelle Bilddaten von der ersten Kamera (18) erhält und die erhaltenen Bilddaten in der Datenbank speichern und mittels der Auswerteeinheit mit bereits gespeicherten Bilddaten von Instrumenten (58, 64) vergleichen und dadurch ein jeweiliges Instrument (58, 64) optisch identifizieren kann. Die Erfindung betrifft ferner ein entsprechendes Verfahren zum Zusammenstellen von Instrumentensets.

## Beschreibung

Die vorliegende Erfindung betrifft eine Vorrichtung zum Zusammenstellen von medizinischen Instrumentensets, mit einer Datenverarbeitungsanlage und einer Feinerkennungseinheit, wobei die Datenverarbeitungsanlage eine Anzeigeeinheit, eine Datenbank, eine erste Schnittstelle und eine Auswerteeinheit aufweist, und wobei die Feinerkennungseinheit eine erste Auflage zum Auflegen der medizinischen Instrumente eines Instrumentensets und eine erste Kamera aufweist, und die erste Kamera so angeordnet und ausgerichtet ist, dass sie Bilddaten von zu erkennenden medizinischen Instrumenten auf der ersten Auflage aus zumindest einer Perspektive erfassen kann, und wobei die Datenverarbeitungsanlage so ausgestaltet ist, dass sie über die erste Schnittstelle Bilddaten von der ersten Kamera erhält und die erhaltenen Bilddaten in der Datenbank speichern und mittels der Auswerteeinheit mit bereits gespeicherten Bilddaten von medizinischen Instrumenten vergleichen und dadurch ein jeweiliges medizinisches Instrument optisch identifizieren kann. Ferner betrifft die vorliegende Erfindung ein Verfahren zum Zusammenstellen von medizinischen Instrumentensets.

In der Medizin, speziell bei medizinischen Operationen, werden für medizinische Eingriffe je nach Art und Umfang des Eingriffs verschiedene medizinische Instrumente benötigt. Diese medizinischen Instrumente werden in Abhängigkeit von dem jeweiligen Eingriff zu einem kompletten Instrumentenset zusammengefasst. Dieses Instrumentenset enthält alle für den geplanten Eingriff notwendigen medizinischen Instrumente, im Folgenden häufig einfach als Instrumente bezeichnet.

Um Infektionen bei diesen medizinischen Eingriffen zu vermeiden, müssen die benötigten medizinischen Instrumente vor dem Eingriff gereinigt und sterilisiert werden. Dies geschieht durch Reinigen und Sterilisieren eines bereits zusammengestellten Instrumentensets. Hierzu wird dieses Instrumentenset vorzugsweise auf ein sogenanntes Instrumentensieb gelegt. Dieses Instrumentensieb ist geeignet, direkt in Spülmaschinen oder Sterilisationsvorrichtungen samt der medizinischen Instrumente des Instrumentensets überführt sowie anschließend wieder entnommen zu werden. In diesem Instrumentensieb sind dann für den jeweiligen anstehenden Eingriff alle notwendigen Instrumente bereitgelegt. Dabei kann ein solches Instrumentensieb mit den Instrumentensets nicht nur von einer zur anderen Eingriffsart sondern auch von Krankenhaus zu Krankenhaus oder sogar von einer zur anderen Abteilung in einem Krankenhaus variieren. Neben der Zusammenstellung des jeweiligen Instrumentensets in einem Instrumentensieb ist hierbei auch die genaue Anordnung der Instrumente in dem jeweiligen Instrumentensieb, die sogenannte Packweise, wichtig und zu beachten. Letztendlich ergibt sich so für jede Abteilung eine zu berücksichtigende individuelle Instrumentensiebkonstellation.

Praktisch wird dies so bewerkstelligt, dass für jedes Instrumentensieb eine spezifische Packliste vorliegt. Diese Packliste enthält neben den Informationen, welche Instrumente zu dem jeweiligen Instrumentenset gehören, auch Informationen zu der Packweise der Instrumente im Instrumentensieb. Anhand dieser Packliste erfolgt dann das Bestücken oder Packen des Instrumentensiebs durch einen Packer in einer Sterilisationsabteilung. Dabei werden die gereinigten Instrumente normalerweise zunächst auf die Funktionsfähigkeit geprüft und anschließend gemäß der Packliste in die Instrumentensiebe gelegt. Diese werden dann in speziellen Behältern sterilisiert.

Um Vorgänge dieser Art in Krankenhäusern effektiv zu gestalten, werden diese Instrumentensets zumindest für Standardeingriffe als auch für geplante Eingriffe jeweils für den Tag oder sogar mehrere Tage im Voraus auf diese Weise vorbereitet. Dadurch sind diese Instrumentensiebe dann in steriler Form bereits vorrätig und brauchen für die jeweiligen anstehenden Eingriffe und Operationen nur noch aus einem Lagerplatz entnommen zu werden. Um die Effektivität und die Kostenersparnis darüber hinaus zu optimieren, wird es heutzutage insbesondere in kleineren Krankenhäusern so gehandhabt, dass die Reinigungs- und auch Sterilisationsvorgänge an externe Firmen abgegeben werden.

Um den Vorteil dieser Optimierungen nicht dadurch einzubüßen, dass zusätzlich benötigte Instrumente nachträglich beschafft werden müssen, da ein Instrumentensieb unvollständig gepackt war, ist es notwendig, dass die Instrumentensiebe sorgfältig und mit allen benötigten medizinischen Instrumenten, d.h. vollständig gepackt, vorbereitet und sterilisiert werden. Das Fehlen eines Instruments ist insbesondere während einer Operation gravierend, da solche Instrumente nachsterilisiert oder aus anderen Quellen entnommen und in den Operationsbereich eingeschleust werden müssen.

Dies bedeutet, dass sowohl in Sterilisationsabteilungen von Krankenhäusern als auch in externen Firmen die jeweils mit der Zusammenstellung der Instrumentensets beauftragten Personen stets sorgfältig und aufmerksam bei dem Bestücken der Instrumentensiebe arbeiten müssen, um Packfehler zu vermeiden.

Um bei diesen Vorgängen einen besseren Überblick und eine Kontrolle zu behalten, schlägt z.B. die DE 196 14 719 A1 vor, die entsprechenden Instrumente mit Kennzeichnungen zu versehen, die für eine Datenverarbeitungsanlage lesbar sind. Diese Kennzeichnungen können z.B. Barcodes oder Matrixcodes sein, oder auch in Form von normal lesbaren Zeichen vorliegen. Mit einem geeigneten Lesegerät kann eine Bedienungsperson dann diese Bar- bzw. Matrixcodes oder Beschriftungen in den Computer einlesen, der dann wiederum erkennt, um welches Instrument es sich handelt. So ist es damit beispielsweise möglich, eine Packliste für ein Instrumentenset in der Datenverarbeitungsanlage zu hinterlegen. Diese wird dann von einer Bedienungsperson abgearbeitet und die Instrumente zusammengestellt, woraufhin anschließend ein Einlesen der Kennzeichnungen der Instrumente in die Datenverarbeitungsanlage erfolgt. Diese gleicht daraufhin die so anhand der Kennzeichnungen erkannten Instrumente mit den Vorgaben der Packliste ab. Fehlt ein Instrument oder ist ein Instrument falsch von der Bedienungsperson gepackt worden, weist die Datenverarbeitungsanlage dann die Bedienungsperson auf diesen Fehler hin.

Trotz der erkennbaren Vorzüge hat diese vorgeschlagene Möglichkeit diverse Nachteile. Zum einen muss jedes Instrument einzeln eingelesen werden, was einen recht großen Zeitaufwand darstellt und auch nicht gestattet, die Packweise zu überprüfen. Hinzu kommen noch mögliche Fehler beim Einlesen der Kennzeichnungen der medizinischen Instrumente, die ein Wiederholen des Einlesevorgangs notwendig machen. Gerade bei einer Vielzahl von medizinischen Instrumenten wird sich zwar die Verwendung von Matrixcodes anbieten, da diese auf einer bestimmten Fläche im Vergleich zu bspw. Barcodes eine größere Kapazität bieten. Demgegenüber haben Matrixcodes aber den Nachteil, dass sie häufiger Lesefehlern unterliegen als andere vergleichbare Codierungen.

Dies wird zusätzlich dadurch erschwert, dass jedwede Kennzeichnung auf solchen medizinischen Instrumenten mit der Zeit fehlerhaft wird oder verblasst, da die medizinischen Instrumente in der Regel zahlreiche Sterilisationsdurchgänge in ihrer Lebensdauer durchlaufen. Aufgrund der Reinigung mit aggressiven Reinigungs- und Sterilisationsmitteln werden die entsprechenden Kennzeichen mit der Zeit immer schwächer und somit letztendlich nicht mehr lesbar.

Außerdem kann es bei komplexeren Instrumenten, die für den Sterilisationsvorgang demontiert und anschließend wieder remontiert werden, schwierig sein, auf alle Einzelteile eine entsprechende Kennzeichnung anzubringen. Dies ist vor allem bei sehr kleinen Instrumenten und Instrumententeilen der Fall.

Neben den Problemen des Einlesens ist auch das Kennzeichnen der Instrumente an sich zeit- und kostenaufwendig. Hierfür muss in der Regel eine Gravur auf dem Instrument vorgenommen werden. Das wiederum erfordert meist eigene technische Hilfsmittel.

US 2011/0005342 A1 beschreibt eine Vorrichtung zur automatisierten Verarbeitung von medizinischen Instrumenten. Darin werden die Instrumente zur Reinigung und/oder zum Verpacken verarbeitet, d.h. insbesondere sortiert. Hierzu ist eine Identifikation der medizinischen Instrumente im System vorgesehen. Dies geschieht durch Aufnahme von Bilddaten mit einer Kamera und Vergleich dieser Bilddaten mit Bilddaten in einer Datenbank. Daraufhin können die Instrumente noch auf mechanische Fehler untersucht werden und werden dann in Behältern abgelegt, die dann der Vorrichtung zur weiteren Verwendung entnommen werden können.

Hierbei basiert die gesamte Prozedur zum Bereitstellen der Behälter auf der korrekten Erkennung mit der Kamera und der korrekten Arbeitsweise der Vorrichtung. Treten hierbei Packfehler auf, wie sie zuvor bereits beschrieben wurden, führt dies ebenfalls zu den eingangs genannten Problemen.

US 4,943,939 beschreibt eine Vorrichtung zum Bereitstellen und Kontrollieren von medizinischen Instrumenten. Dazu weist die Vorrichtung einen Spender von Instrumenten auf und hat zudem einen modifizierten Ablagetisch, einen sogenannten "Mayo stand". Dieser Ablagetisch weist in einer Ausgestaltung eine Videoaufzeichnungseinheit auf, mit der digitale Bilddaten von den auf dem Ablagetisch angeordneten medizinischen Instrumenten erfasst werden. Zur Kontrolle der in einer Operation verwendeten Instrumente, werden kontinuierliche Aufzeichnungen des Ablagetisches, bzw. der darauf angeordneten Instrumente gemacht und mit vorherigen Bildern verglichen.

Diese Vorrichtung ist somit für eine Überwachung der verwendeten Instrumente ausgestaltet. Eine Identifikation, insbesondere von Instrumentensets zur weiteren Verwendung in einer Operation ist nicht vorgesehen. Entsprechend ist auch dieses System anfällig für die zuvor genannten Erkennungsfehler, die in den unerwünschten Packfehlern resultieren.

Der vorliegenden Erfindung liegt daher die Aufgabe zugrunde, eine Vorrichtung und ein Verfahren bereitzustellen, das zum einen den Packprozess für die angesprochenen Instrumentensets optimiert und insbesondere verlässlich macht, zum anderen aber den Zeit- und Kostenaufwand für die Erkennung der medizinischen Instrumente gering hält und die Anfälligkeit und Probleme im Zusammenhang mit der Beständigkeit und Möglichkeit der Kennzeichen der Instrumente verringert.

Diese Aufgabe wird zum einen durch eine Vorrichtung zum Zusammenstellen von medizinischen Instrumentensets der eingangs genannten Art gelöst, die ferner eine Groberkennungseinheit aufweist, wobei die Groberkennungseinheit eine Auflage aufweist, auf die die Instrumente nach der optischen Identifizierung in der Feinerkennungseinheit zu einem Instrumentenset zusammengeführt werden, und wobei die Auflage der Groberkennungseinheit vorzugsweise durch eine zweite Auflage gebildet wird. Hierbei kann, neben der bevorzugten Ausgestaltung der zweiten separaten Auflage, die Auflage der Groberkennungseinheit auch mit der ersten Auflage der Feinerkennungseinheit identisch sein. Die Auflage der Groberkennungseinheit wird somit durch die erste Auflage gebildet. Dabei würden Fein- und Groberkennungseinheit konstruktiv in dieser Hinsicht zusammenliegen.

Diese Ausgestaltung hat insgesamt den Vorteil, dass das erkannte Instrumentenset nun vor der Sterilisation abermals auf eine Auflage gelegt wird, so dass dort ein letzter Vollständigkeitscheck zur Bestätigung stattfinden kann, bevor das Instrumentenset endgültig in die Sterilisation überführt wird. Hierzu werden die Instrumente in aller Regel in einem Instrumentensieb zu einem Instrumentenset zusammengefügt.

Im Hinblick auf die zuvor erwähnte DE 196 14 719 A1 hat diese Verfahrensweise den Vorteil, dass es bei der einzelnen Erkennung von Kennzeichnungen auf den medizinischen Instrumenten dann immer noch dazu kommen kann, dass selbst nach erfolgreicher Erkennung eines solchen medizinischen Instruments die entsprechende Bedienungsperson das Instrument nicht in die Sterilisation bzw. Reinigung überführt. Somit ist das Instrument zwar als vorhanden erkannt, liegt aber tatsächlich nicht im Instrumentensieb vor.

Dies wird hier dadurch unterbunden, dass die durch die Feinerkennungseinheit erkannten Instrumente in ihrer Gesamtheit zusammengelegt werden und von diesem zusammengelegten Instrumentenset eine Vollständigkeitsüberprüfung durch die Groberkennungseinheit stattfindet. Erst nach dessen Vollständigkeit wird das komplette Set der weiteren Reinigung bzw. Sterilisation zugeführt.

Im Hinblick auf die US 2011/00025324 A1 und die US 4,943,939 liegt der Vorteil darin, dass durch die zweite grobe Prüfung durch die Groberkennungseinheit ein oder mehrere mögliche Erkennungsfehler von Instrumenten durch die Systeme vor der Verwendung, d.h. vor der Sterilisation oder spätestens vor dem Bereitstellen als einsatzfähiges Instrumentenset, das in einen Operationssaal überbracht werden kann, bemerkt werden können. Dies erhöht somit signifikant die Verlässlichkeit des Systems.

Diese erfindungsgemäße Vorrichtung hat ferner insbesondere gegenüber Systemen die eine Kennzeichnung der Instrumente benötigen den Vorteil, dass es möglich ist, durch die Erfassung der Bilddaten durch eine Kamera im Zusammenhang mit der entsprechend ausgestalteten Datenverarbeitungsanlage eine Erkennung der Instrumente ohne die Notwendigkeit von Kennzeichnungen auf den Instrumenten durchzuführen. Dabei ist es neben der Identifizierung der Instrumente gleichzeitig möglich, deren Anordnung auf der Auflage, also die Packweise, zu überprüfen.

Hierzu wird in der Regel eine Einlernphase für die entsprechenden Instrumente vorausgehen, in der die jeweiligen Instrumente von Kamera und Datenverarbeitungsanlage erfasst und erkannt werden und ihnen dabei eine entsprechende Kennzeichnung zugeordnet wird. Dazu wird diese in der Datenbank der Datenverarbeitungsanlage zusammen mit den Referenzbilddaten abgelegt. Hierbei kann auch berücksichtigt werden, dass einzelne Instrumente eine unterschiedliche Bilddarstellung aufweisen, je nachdem wie man sie auf die Auflage legt. Als Beispiel seien hier gekrümmte Scheren oder Klemmen erwähnt, deren Bilddarstellungen, wie sie die Kamera erfasst, je nach aufgelegter Seite unterschiedlich sind. Dies zeigt sich daran, dass sie nicht durch Drehung oder Translation miteinander zur Deckung gebracht werden können. Aus diesem Grund können dann die entsprechenden möglichen Lagepositionen separat eingelernt werden und unter derselben Kennzeichnung in der Datenbank abgelegt werden.

Ferner hat die Vorrichtung den Vorteil, dass nicht jedes Instrument einzeln erkannt werden bzw. eingelesen werden muss, sondern die Gesamtheit aller Instrumente eines Instrumentensets auf der Auflage ausgebreitet werden kann. Die Vorrichtung kann daraufhin ein Gesamtbild der Auflage mit den entsprechenden Instrumenten über die Kamera erfassen und die erfassten Bilddaten an die Datenverarbeitungsanlage weitergeben. Die Auswerteeinheit der Datenverabeitungsanlage kann anschließend mit Hilfe entsprechender Objekterkennungsalgorithmen die einzelnen Instrumente erkennen und diese mit der Packliste für das zu packende Instrumentenset abgleichen. Beispiele für solche Algorithmen und Methoden der Objekterkennung sind Korrelationsmethoden bei denen mittels Korrelation bestimmt wird, wo auf dem Bild sich ein eintrainiertes Objekt befindet. Hierzu werden die eintrainierten Referenzbilder des Objekts iterativ über das erfasste Gesamtbild bewegt und für jede Position bestimmt, welchen Korrelationswert ein jeweiliges Referenzbild an der betreffenden Position im Gesamtbild hat. Das Maximum der Korrelation über alle verschiedenen Referenzbilder zeigt dann an, welches Objekt sich an welcher Position im Gesamtbild und somit auf der Auflage befindet. Dabei kann die Korrelation beispielsweise anhand der jeweiligen Bildkanalwerte ermittelt werden. Alternativ ist auch eine kantenbasierte Vergleichsmethode, bei der also anstatt der Bildkanalwerte die Kanten der Objekte als Vergleichswerte dienen, denkbar.

Als alternative Erkennungsmethode sei hier exemplarisch auch die generalisierte Hough-Transformation genannt. Dabei werden zunächst verschiedene Suchobjekte anhand ihrer Kanteninformationen eintrainiert und für jedes Referenzobjekt die Richtungsvektoren zwischen dem Objektschwerpunkt und den Kantenpixeln hinterlegt. Die Erkennung erfolgt dann anhand der Kantenpixel und den dazu gespeicherten Lageverschiebungen zum Objektschwerpunkt. Als Alternative hierzu werden bei der Methode der Objektkategorisierung Teilregionen der Objekte eintrainiert und die jeweiligen Richtungsvektoren zum Objektschwerpunkt hinterlegt. Die Erkennung erfolgt dann entsprechend über die Erkennung der Teilregionen im Gesamtbild und ihrer Lage zum Objektschwerpunkt.

Zum Zwecke der Beschleunigung der Objekterkennung ist ferner denkbar, die Erkennung auf das Auffinden von Schlüsselmerkmalen zu beschränken. Unter dem Begriff Schlüsselmerkmale sind im Rahmen dieser Erfindung optische Instrumentenmerkmale zu verstehen. Diese können beim Eintrainieren eines Instruments automatisch vorgegeben oder auch mittels automatischer Algorithmen für jedes Instrument berechnet werden. Hierbei handelt es sich in aller Regel um auffällige, markante und/oder gut sichtbare Bereiche des jeweiligen Instruments. Aus diesem Grund ist es auch möglich, dass insbesondere ein erfahrener Packer selber beim Eintrainieren angibt, welche Instrumentenbereiche Schlüsselmerkmale darstellen, die sie von anderen, sehr ähnlich aufgebauten Instrumenten unterscheiden. Hierbei kann es sich auch um kleine Details, wie z.B. kleine Instrumentenbestandteile, verschiedene Oberflächenriffelungen, Einbuchtungen, kleine Nuten oder dergleichen handeln. Auch extra für diesen Zweck aufgebrachte Markierungen oder andere Kennzeichnungen sind denkbar. Die alternative oder zusätzliche automatische Bestimmung von Schlüsselmerkmalen erfolgt beispielsweise über sogenannte Interest-Operatoren, wie z.B. dem Förstner-Operator oder dem Moravec-Operator.

Um eine exakte Erkennung der einzelnen Instrumente zu ermöglichen, sollten diese vorzugsweise überlagerungsfrei auf der ersten Auflage nebeneinander angeordnet sein. Hiervon kann jedoch bei der Erkennung anhand von Schlüsselmerkmalen abgewichen werden.

In einer weiteren Ausgestaltung der Vorrichtung weist die erste Auflage einen kontrastverstärkenden Hintergrund auf, der so angeordnet ist, dass er aus der jeweiligen Kameraperspektive hinter einem zu erkennenden Instrument liegt.

Die Verwendung eines kontrastverstärkenden Hintergrunds hat den Vorteil, dass bei den zuvor beispielhaft erwähnten Methoden der Objekterkennung eine Zuordnung der Bilddaten zu entsprechenden Instrumententypen vereinfacht wird. Das liegt daran, dass die Abgrenzung zwischen vermeintlichem Instrument und Hintergrund deutlicher und somit für einen entsprechenden Objekterkennungsalgorithmus eindeutiger wird. Dies ist insbesondere bei den vorliegenden medizinischen Instrumenten wichtig, da diese zumeist aus Medizinstahl gefertigt sind, der sich mit seiner grau glänzenden Oberfläche nur schwer von regulären, also weißen Untergründen abhebt. Ferner ist auch bei einem solchen eindeutig unterschiedlichen Hintergrund ein zuverlässiges Herausfiltern der Bildinformation des Hintergrunds möglich. Ein solcher kontrastverstärkender Hintergrund kann beispielsweise eine blaue Fläche sein.

In einer weiteren Ausgestaltung der Erfindung ist die erste Kamera in ihrer Position veränderbar angeordnet, so dass sie Bilddaten aus mindestens zwei Perspektiven erfassen kann.

Wenn hierbei und im Folgenden von "Perspektive" die Rede ist, so ist hierunter die Orientierung einer jeweiligen Kamera zu einem jeweiligen Instrument in Hinblick auf Azimutwinkel und Polarwinkel gemeint. Dabei führt eine Änderung der Perspektive zu einer Änderung dieser Orientierung, indem zumindest einer dieser beiden Winkel, die zusammen mit dem Abstand zum Ursprung eines Kugelkoordinatensystems die Position eines Objekts in einem solchen System eindeutig festlegen, verändert wird.

Durch diese Anordnung ist es vorteilhafterweise möglich, dass die Kamera von einer Anordnung von Instrumenten auf der Auflage Bilddaten aus mehreren Orientierungen erfassen kann. Dadurch können die Eindeutigkeit und die Verlässlichkeit der Erkennung der medizinischen Instrumente erhöht werden, da zusätzliche Aufnahmen aus anderen Perspektiven die Anzahl der Informationen erhöhen. Dies ist insbesondere bei komplexeren medizinischen Instrumenten hilfreich, die sich häufig in kleinen Details unterscheiden, die aus nur einer einzigen Orientierung nicht zu erkennen sein könnten.

In einer weiteren Ausgestaltung der Erfindung weist die erste Auflage eine transparente Grundfläche auf, auf die die zu erkennenden Instrumente aufgelegt werden.

Diese Ausgestaltung hat den Vorteil, dass das Erfassen der Bilddaten nicht auf eine Seite der Auflage beschränkt ist. So ist es bei dieser Ausgestaltung auch möglich, Bilddaten von unterhalb der Auflage zu erfassen. Für die Ausgestaltung der transparenten Grundfläche kommen vorzugsweise Glas oder transparente Kunststoffe, wie z.B. Plexiglas, in Frage.

In einer weiteren Ausgestaltung der Erfindung weist die Feinerkennungseinheit ferner eine zweite Kamera auf, die so angeordnet und ausgerichtet ist, dass sie Bilddaten von zu erkennenden Instrumenten auf der ersten Auflage aus zumindest einer weiteren Perspektive erfassen kann, wobei die Datenverarbeitungsanlage so ausgestaltet ist, dass sie über eine Schnittstelle zusätzlich Bilddaten von der zweiten Kamera erhält und die erhaltenen Bilddaten in der Datenbank speichern und mittels der Auswerteeinheit mit bereits gespeicherten Bilddaten von Instrumenten vergleichen und dadurch ein jeweiliges Instrument optisch identifizieren kann, wobei die erste Kamera auf der einen und die zweite Kamera auf der anderen gegenüberliegenden Seite der Grundfläche angeordnet ist, wobei vorzugsweise die zweite Kamera in ihrer Position veränderbar angeordnet ist, so dass sie Bilddaten aus zumindest zwei Perspektiven erfassen kann.

Das Vorsehen einer zweiten Kamera hat den Vorteil, dass die zuvor beschriebene Möglichkeit der Erkennung und Erfassung der Bilddaten von der Unterseite ohne größeren Aufwand hinsichtlich der Verstellung der ersten Kamera möglich ist. Hierzu wird lediglich ein zuvor beschriebenes Verschieben des kontrastverstärkenden Hintergrunds benötigt, je nachdem welche Kamera Bilddaten erfasst. Die Schnittstelle, über die die Datenverarbeitungsanlage die Bilddaten von der zweiten Kamera erhält, kann dabei sowohl eine zu den zuvor gemachten Ausführungen analoge zusätzliche Schnittstelle als auch die zuvor genannte erste Schnittstelle sein.

Die so erfassten Bilddaten von Ober- und Unterseite der medizinischen Instrumente können zusammen in der Auswerteeinheit der Datenverarbeitungsanlage analysiert und ausgewertet werden, was die Trefferquote der Objekterkennung der Instrumente deutlich erhöht. Dabei ist es vorzugsweise so, dass bei einer erfolgreichen Erkennung eines Instruments anhand der Bilddaten von einer Seite auf die Auswertung oder sogar auf die Erfassung der Bilddaten von der anderen Seite verzichtet werden kann.

In einer weiteren Ausgestaltung der Erfindung kann der kontrastverstärkende Hintergrund zwischen mehreren Positionen verschiebbar angeordnet sein, wobei die mehreren Positionen auf jeweils gegenüberliegenden Seiten der transparenten Grundfläche liegen.

Diese Ausgestaltung hat den Vorteil, dass die Instrumente in optischer Hinsicht von allen Seiten zugänglich sind. So können durch diese bevorzugte Ausgestaltung zum einen Bilddaten von der Oberseite und anschließend durch Entfernen des kontrastverstärkenden Hintergrunds von der Unterseite erfasst werden. Das jeweilige Anordnen des kontrastverstärkenden Hintergrunds gestattet dabei in jeder Kameraperspektive die gute Erkennbarkeit der Instrumente in der Objekterkennung, wie sie zuvor beschrieben wurde. Dies ist insbesondere bei der Verwendung von zwei Kameras, die jeweils auf einer Seite der Grundfläche angeordnet sind, von Vorteil.

Die Verschiebung des kontrastverstärkenden Hintergrunds kann vorzugsweise motorgetrieben und automatisch geschehen.

In einer weiteren Ausgestaltung der Erfindung weist die Vorrichtung zumindest zwei kontrastverstärkende Hintergründe auf, die jeweils so auf den gegenüberliegenden Seiten der transparenten Grundfläche angeordnet sind, dass jeweils zumindest ein kontrastverstärkender Hintergrund aus einer jeweiligen Kameraperspektive hinter dem zu erkennenden Instrument liegt.

Bei dieser Ausgestaltung wird ein jeweiliger kontrastverstärkender Hintergrund auf jeder Seite der transparenten Grundfläche bereitgestellt. Somit wird für jede Kameraperspektive, unabhängig von der momentanen Anordnung der Kamera mit Bezug auf die Grundfläche, die gute Erkennbarkeit der Instrumente in der Objekterkennung, wie sie zuvor beschrieben wurde, gewährleistet. Dies ist insbesondere bei der Verwendung von zwei Kameras, die jeweils auf einer Seite der Grundfläche angeordnet sind, von Vorteil, da so in einem kurzen zeitlichen Abstand, vorzugsweise gleichzeitig, mit beiden Kameras Aufnahmen gemacht werden können. Damit wird die gesamte Objekterkennung durch die zusätzlichen Bildinformationen optimiert, gleichzeitig aber nicht verlangsamt.

In einer weiteren Ausgestaltung der Erfindung weist die Auflage der Groberkennungseinheit eine Waage und die Datenverarbeitungsanlage eine zweite Schnittstelle auf, wobei die Datenverarbeitungsanlage ferner so ausgestaltet ist, dass sie über die zweite Schnittstelle Gewichtsdaten des aufgelegten Instrumentensets von der Waage erhält und die erhaltenen Gewichtsdaten in der Datenbank speichern und mittels der Auswerteeinheit mit bereits gespeicherten Gewichtsdaten der optisch identifizierten Instrumente vergleichen kann.

Diese Ausgestaltung hat zum einen den Vorteil, dass so nun noch eine zusätzliche Kontrolle des kompletten Instrumentensets mit Hilfe eines anderen Erkennungsverfahrens erfolgt. Dabei hat die Verwendung einer Waage den Vorteil, dass im Gegensatz zu einer Einzelerkennung der medizinischen Instrumente die Art des Auflegens der medizinischen Instrumente unerheblich ist, da dies nicht das Gewicht beeinflusst. So können die medizinischen Instrumente sich überlagern, ohne dass dies eine Beeinträchtigung dieser Erkennungsart hervorruft. Dies spart zum einen Zeit und auch Platz.

Ferner hat die Verwendung einer Waage den Vorteil, dass komplexere Instrumente, die aus mehreren zusammengebauten Teilen bestehen, somit noch einmal auf Vollständigkeit überprüft werden können. Dies vermag bei einer optischen Erkennung der äußeren Charakteristika nicht möglich zu sein, wenn z.B. ein einzelnes Teil aus dem Inneren des Instruments fehlt. Ist dies der Fall, würde dies aber bei der Bestimmung des Gewichts, selbst des Gesamtgewichts des Instrumentensets, sich dahingehend auswirken, dass das Gesamtgewicht des Instrumentensets von dem Sollgewicht abweicht.

Das Sollgewicht des Instrumentensets kann dabei vorzugsweise durch die Datenverarbeitungsanlage individuell berechnet werden. Hierzu kann die Datenverarbeitungsanlage auf die Gewichtsinformation der einzelnen Instrumente zurückgreifen, die Bestandteil des zu packenden Instrumentensets sind. Alternativ kann auch das Gesamtgewicht eines jeweiligen Instrumentensets in Zusammenhang mit der Packliste in der Datenbank der Datenverarbeitungsanlage hinterlegt sein.

Die hinterlegten Referenzgewichte der jeweiligen Instrumente oder auch der gesamten Instrumentensets können entweder nach Herstellervorgaben in die Datenbank der Datenverarbeitungsanlage übernommen werden oder in einer separaten Einlernphase erfasst werden. Hierbei ist es denkbar, dass mehrere Wägevorgänge für ein Instrument oder ein jeweiliges komplettes Instrumentenset stattfinden können, über die ein Mittelwert gebildet wird und ebenfalls eine entsprechende Abweichung mit hinterlegt werden kann. Dadurch wäre es dann möglich, dass für die entsprechenden Gewichte der Instrumente bzw. Instrumentensets aufgrund von ermittelten Standardabweichungen ein gewisser Toleranzbereich in der Datenbank mit abgelegt wird, so dass nicht bereits ein minimaler Gewichtsunterschied zu einem Fehler führt, sondern die Datenverarbeitungsanlage erst ab Überschreiten des Toleranzbereichs einen Fehler für das zusammengestellte Instrumentenset meldet.

In einer weiteren Ausgestaltung der Erfindung weist die Groberkennungseinheit eine Kamera auf, die so angeordnet und ausgerichtet ist, dass sie Bilddaten eines Instrumentensets auf der Auflage der Groberkennungseinheit aus zumindest einer Perspektive erfassen kann, und die Datenverarbeitungsanlage so ausgestaltet, dass sie über eine Schnittstelle Bilddaten von der Kamera der Groberkennungseinheit erhält und die erhaltenen Bilddaten in der Datenbank speichern und mittels der Auswerteeinheit mit bereits gespeicherten Bilddaten von Instrumenten vergleichen und dadurch die jeweiligen Instrumente optisch identifizieren und die Vollständigkeit des Instrumentensets überprüfen kann, wobei die Kamera der Groberkennungseinheit vorzugsweise durch eine weitere Kamera gebildet wird und die Datenverarbeitungsanlage eine dritte Schnittstelle aufweist und so ausgestaltet ist, dass sie über die dritte Schnittstelle Bilddaten von der weiteren Kamera erhält. Hierbei ist es neben dieser bevorzugten Ausgestaltung der weiteren Kamera ebenfalls möglich, dass die Kamera der Groberkennungseinheit identisch ist mit einer Kamera der Feinerkennungseinheit.

Diese Ausgestaltung hat insgesamt den Vorteil, dass hierbei bei der Groberkennung des Instrumentensets ebenfalls wieder ein schnelles optisches Verfahren zum Einsatz kommt, welches ebenfalls die individuellen Instrumente des Instrumentensets erkennen kann.

Da es bei einem solchen Instrumentenset auf der Auflage der Groberkennungseinheit auch zu Überlagerungen von Instrumenten kommen kann, wird hierbei vorzugsweise eine Objekterkennung anhand von Schlüsselmerkmalen vorgenommen. Somit brauchen nicht alle Instrumente komplett erkennbar sein. Dabei kann vorgegeben werden, wie viele der hinterlegten Schlüsselmerkmale erkannt werden müssen. Daneben ist die Erkennung von Schlüsselmerkmalen bei der Identifizierung der einzelnen Instrumente besonders auch bei der Verwendung von Instrumentensieben, in denen die Instrumente zu den jeweiligen Instrumentensets zusammengestellt werden, von Vorteil. Das liegt daran, dass die Instrumentensiebe, wie auch die Instrumente selber, aus Metall gefertigt sind. Eine optische Erkennung wird somit generell dadurch erschwert, dass Hintergrund, also Instrumentensieb, und zu erkennendes Objekt, also Instrument, aus dem gleichen Material gefertigt sind und sich somit optisch ähneln. Hierbei kann durch die Konzentration auf Schlüsselmerkmale jedoch die notwendige Unterscheidbarkeit erreicht werden.

Neben der Möglichkeit, dieses zweite optische Verfahren allein zu verwenden, genauso wie das zuvor beschriebene Wägeverfahren allein zu verwenden, besteht auch die Möglichkeit, in einer bevorzugten Ausgestaltung die beiden Verfahren miteinander zu kombinieren.

Eine solche Vorrichtung hätte dann zum einen die zuvor beschriebene Waage zur Bestimmung des Gesamtgewichts des Instrumentensets als auch eine (weitere) Kamera zur Identifizierung der aufgelegten Instrumente anhand von Schlüsselmerkmalen.

Damit werden die Vorteile des individuellen Erkennens der Instrumente mit der (weiteren) Kamera mit der Möglichkeit kombiniert, auch fehlende Innenteile der Instrumente aufgrund der Gewichtsabweichung zu bemerken.

Im Übrigen ergeben sich auch für die Vorrichtung mit der Kamera, alleine oder in Kombination mit der Waage, die zuvor im Zusammenhang mit der Ausgestaltung mit der Waage beschriebenen Vorteile, insbesondere, dass durch eine solche Vorrichtung noch eine abschließende Kontrolle der Vollständigkeit des Instrumentensets vor der Sterilisation und Reinigung erfolgen kann.

In einer weiteren Ausgestaltung der Erfindung ist die Kamera der Groberkennungseinheit in ihrer Position veränderbar angeordnet, so dass sie Bilddaten aus zumindest zwei Perspektiven erfassen kann.

Dies hat, wie auch zuvor schon beschrieben, den Vorteil, dass so die Zuverlässigkeit des Objekterkennungsverfahrens dadurch erhöht wird, dass mehr Bildinformationen bzw. Bilddaten vorhanden sind. Dies ist insbesondere dann von Vorteil, wenn wie hier eine Überlagerung von Instrumenten vorliegen kann und die Vorrichtung es somit gestattet, auch Bildinformationen von Instrumenten zu erhalten, die entweder ganz oder teilweise aus der einen Perspektive von anderen Instrumenten überlagert werden.

In einer weiteren Ausgestaltung weist die Auflage der Groberkennungseinheit ein Instrumentensieb auf, in das die Instrumente gelegt und anschließend als vollständiges Instrumentenset sterilisiert werden können.

Diese Ausgestaltung der Vorrichtung hat den Vorteil, dass auf diese Weise nur noch ein bereits gepacktes Instrumentensieb von der Auflage der Groberkennungseinheit abgenommen werden muss, ohne dass ein weiteres Übertragen der Instrumente von einer Auflage zur Sterilisation oder Reinigung als einzelne Instrumente notwendig ist. Auf diese Weise wird somit die Vollständigkeit des Instrumentensets so bestimmt, wie es auch in dem Instrumentensieb landet, das dann direkt in Reinigungs- bzw. Sterilisationseinrichtungen überführt werden kann.

Ferner wird die eingangs genannte Aufgabe durch ein Verfahren zum Zusammenstellen von medizinischen Instrumentensets mit einer Vorrichtung der vorgenannten Art gelöst, das folgende Schritte aufweist:
a) Auflegen von zumindest einem Instrument auf die erste Auflage der Feinerkennungseinheit,
b) Erfassen der Bilddaten durch zumindest die erste Kamera,
c) Weitergabe der Bilddaten an die Datenverarbeitungsanlage,
d) Vergleich der Bilddaten mit den in der Datenbank gespeicherten Referenzbildern für die Instrumente des zusammenzustellenden Instrumentensets,
e) Informieren des Benutzers über die Anzeigeeinheit über fehlende oder überflüssige Instrumente bzw. über die Vollständigkeit des Instrumentensets.

Dieses Verfahren hat den Vorteil, dass der Benutzer nicht jedes Instrument einzeln erkennen lassen muss, sondern einen Satz von Instrumenten auf die erste Auflage legt. Dieser Satz kann bereits das Instrumentenset bilden und besteht vorzugsweise aus einer Vielzahl von Instrumenten. Nach Abgleich der erfassten Bilddaten mit den in der Datenbank gespeicherten Referenzbildern, die durch eine zuvor beschriebene Einlernphase erhalten werden können, erhält der Benutzer so auf einfache Weise nach kurzer Zeit eine Information, welche Instrumente fehlen, welche überflüssig oder falsch sind bzw. ob das Instrumentenset so in der aufgelegten Form vollständig ist und sterilisiert werden kann.

Im Übrigen ergeben sich für das zuvor beschriebene Verfahren die entsprechenden im Zusammenhang mit der Vorrichtung zuvor genannten Vorteile.

In einer weiteren Ausgestaltung des erfindungsgemäßen Verfahrens werden im Schritt b) die Bilddaten durch die erste und zweite Kamera erfasst.

Dies hat den Vorteil, dass auf diese Weise Bilddaten von zumindest zwei Seiten zur Verfügung stehen, wodurch zum einen das Objekterkennungsverfahren zuverlässiger wird und aufgrund der deutlich größeren Menge an Informationen auch schneller ein verlässliches Ergebnis liefert.

In einer weiteren Ausgestaltung des erfindungsgemäßen Verfahrens erfasst zumindest eine Kamera Bilddaten aus zumindest zwei Perspektiven, indem die jeweilige Kameraposition verändert wird.

Auch diese Ausgestaltung des Verfahrens hat den Vorteil, dass die Informationsmenge erhöht wird. Somit liegen abermals mehr Bilddaten derselben Anordnung an Instrumenten vor, was die Zuverlässigkeit der Objekterkennung erhöht und die erfolgreiche Objekterkennung beschleunigt.

Dabei ist es vorzugsweise so, dass die Position der Kamera automatisch verändert wird. Dies kann u.a. durch Ansteuerung durch die Datenverarbeitungsanlage geschehen.

In einer weiteren Ausgestaltung des erfindungsgemäßen Verfahrens weist dieses ferner folgende Schritte auf:
f) Ermitteln des Gewichts des Instrumentensets mit der Waage,
g) Übermitteln des Gewichts an die Datenverarbeitungsanlage,
h) Vergleichen des Gewichts mit einem Sollgewicht des Instrumentensets,
i) Informieren des Benutzers über Vollständigkeit oder Fehler des Instrumentensets.

Diese Verfahrensschritte haben den Vorteil, dass dadurch nach der zuvor beschriebenen individuellen Identifizierung der Instrumente und der Richtigkeit dieser Instrumente in Hinblick auf das zusammenzustellende Instrumentenset noch einmal abschließend die Vollständigkeit des Instrumentensets vor der Sterilisation überprüft werden kann.

Dazu wird sich hier wie beschrieben einer Groberkennung bedient, was in diesem speziellen erfindungsgemäßen Fall durch eine Waage realisiert wird. Dies hat den Vorteil, dass die Groberkennung sehr schnell durchgeführt werden kann. Ferner kann so auch eine Vollständigkeit im Hinblick auf zusammenzubauende Instrumente noch einmal abschließend überprüft werden. Dies ist der äußeren Erscheinung des Instruments nicht immer zu entnehmen. Im Übrigen wird hierzu auch auf die zuvor bereits ausgeführten Vorteile bei der Verwendung einer Waage Bezug genommen.

In einer alternativen Ausgestaltung des erfindungsgemäßen Verfahrens weist dieses ferner folgende Schritte auf:
f) Erfassen der Bilddaten des Instrumentensets mit der Kamera der Groberkennungseinheit,
g) Übermitteln der Bilddaten an die Datenverarbeitungsanlage,
h) Vergleichen der Bilddaten mit den in der Datenbank gespeicherten Bilddaten der Instrumente, die zu dem Instrumentenset gehören,
l) Informieren des Benutzers über Vollständigkeit oder Fehler des Instrumentensets,
wobei der Vergleich in Schritt h) vorzugsweise anhand von Schlüsselmerkmalen der Instrumente stattfindet.

Bei dieser Alternative der erfindungsgemäßen Groberkennung wird abermals auf ein optisches Verfahren zurückgegriffen. Dies hat den Vorteil, dass wieder jedes Instrument individuell erkannt wird. Ein fehlendes Instrument kann somit genau bestimmt werden. Um hier zum einen die Geschwindigkeit zu erhöhen als auch dem Problem der sich überlappenden Instrumente auf der Auflage der Groberkennungseinheit Rechnung zu tragen, wird auf den erfassten Bilddaten nach Schlüsselmerkmalen gesucht anstatt ein vollständiges Instrument zu erkennen, wie dies bei der eingangs genannten Feinerkennung der Fall ist.

In einer bevorzugten Ausgestaltung dieses erfindungsgemäßen Verfahrens weist das zuvor genannte Verfahren zwischen den Schritten h) und l) folgende Schritte auf:
i) Ermitteln des Gewichts des Instrumentensets mit der Waage,
j) Übermitteln des Gewichts an die Datenverarbeitungsanlage,
k) Vergleichen des Gewichts mit einem Sollgewicht des Instrumentensets.

Diese bevorzugte Ausgestaltung hat den Vorteil, dass die beiden Groberkennungsverfahren, d.h. das Wägeverfahren und das optische Erkennungsverfahren anhand von Schlüsselmerkmalen miteinander kombiniert werden. Dies erhöht die Verlässlichkeit dieser Groberkennung. Da beide Verfahren parallel zueinander ablaufen können, wird jedoch dabei nicht die Dauer dieses Groberkennungsverfahrens erhöht. Im Übrigen ergänzen sich die zuvor erwähnten Vorteile der Einzelverfahren der Groberkennung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert. Es zeigen:
- Fig. 1: eine schematische perspektivische Darstellung einer erfindungsgemäßen Vorrichtung mit Feinerkennungseinheit,
- Fig. 2: eine schematische Darstellung des Aufbaus einer erfindungsgemäßen Datenverarbeitungsanlage,
- Fig. 3: eine schematische perspektivische Darstellung einer weiteren erfindungsgemäßen Vorrichtung mit einer Kombination aus Feinerkennungseinheit und Groberkennungseinheit, Letztere mit einer Kamera,
- Fig. 4: eine schematische perspektivische Darstellung einer weiteren erfindungsgemäßen Vorrichtung mit einer Kombination aus Feinerkennungseinheit mit Groberkennungseinheit, Letztere mit einer Wage,
- Fig. 5: eine schematische Darstellung einer Datenverarbeitungsanlage für eine Vorrichtung entsprechend der Fig. 4,
- Fig. 6: eine schematische perspektivische Darstellung einer Groberkennungseinheit mit Kamera und Waage,
- Fig. 7: eine schematische Darstellung einer Datenverarbeitungsanlage für eine Gesamtvorrichtung mit einer Groberkennungseinheit entsprechend Fig. 6,
- Fig. 8: eine schematische perspektivische Darstellung einer Kameraanordnung für Fein- und Groberkennungseinheiten mit veränderbarer Perspektive,
- Fig. 9: eine schematische Seitendarstellung einer erfindungsgemäßen Vorrichtung mit Feinerkennungseinheit mit erster und zweiter Kamera und kontrastverstärktem Hintergrund unter dem zu erkennenden Instrument,
- Fig. 10: eine schematische Seitendarstellung einer erfindungsgemäßen Vorrichtung mit Feinerkennungseinheit mit erster und zweiter Kamera entsprechend der Fig. 9, mit kontrastverstärkendem Hintergrund über dem zu erkennenden Instrument,
- Fig. 11: eine schematische Seitendarstellung einer weiteren Ausführungsform einer erfindungsgemäßen Vorrichtung mit einer Feinerkennungseinheit analog zur Vorrichtung aus den Fig. 9 und 10 und zwei kontrastverstärkenden Hintergründen, und
- Fig. 12: eine schematische Seitendarstellung einer weiteren Ausführungsform einer erfindungsgemäßen Vorrichtung mit einer Feinerkennungseinheit analog zur Vorrichtung aus den Fig. 9, 10 und 11, mit ebenfalls zwei kontrastverstärkenden Hintergründen.

Die im Nachfolgenden gezeigten und beschriebenen erfindungsgemäßen Vorrichtungen sind in ihrer Gesamtheit mit den Bezugsziffern 10, 100, 150, 300, 350 und 370 bezeichnet.

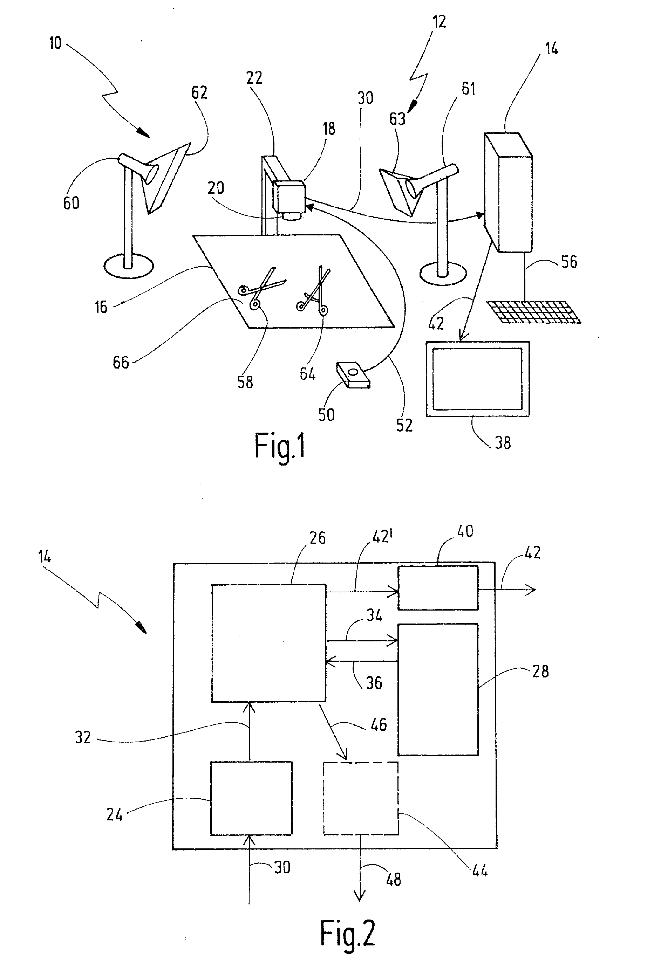

Die in Fig. 1 gezeigte erfindungsgemäße Vorrichtung 10 weist eine Feinerkennungseinheit 12 und eine Datenverarbeitungsanlage 14 auf. Die Feinerkennungseinheit 12 weist eine erste Auflage 16 und eine erste Kamera 18 auf.

Die erste Kamera 18 ist so über der ersten Auflage 16 angeordnet, dass ihr Objektiv 20, und entsprechend somit ihre Perspektive auf die erste Auflage 16 gerichtet ist. Hierzu ist die erste Kamera 18 mittels eines Stativs 22 über der ersten Auflage 16 mit Bezug auf die Darstellung von Fig. 1 angeordnet.

Die Datenverarbeitungsanlage 14 weist ihrerseits eine erste Schnittstelle 24, eine Auswerteeinheit 26 sowie eine Datenbank 28 auf, wie dies in Fig. 2 zu sehen ist. Über die erste Schnittstelle 24 ist die Datenverarbeitungsanlage 14 mit der ersten Kamera 18 verbunden und erhält über diese erste Schnittstelle 24 Bilddaten von der ersten Kamera 18. Dies ist durch einen Pfeil 30 schematisch in Fig. 1 und 2 angedeutet. Die so erhaltenen Bilddaten werden dann von der ersten Schnittstelle 24 an die Auswerteeinheit 26 weitergegeben. Dies ist durch einen Pfeil 32 schematisch angedeutet. Die Auswerteeinheit 26 kann nun mit der Auswertung und Analyse der so erhaltenen Bilddaten direkt beginnen, oder die Daten zunächst in der Datenbank 28 ablegen. Dies ist durch einen Pfeil 34 angedeutet. Zum Zwecke der Auswertung der Daten in der Auswerteeinheit 26 benötigt diese Referenzbilddaten, wie dies im Folgenden noch näher beschrieben wird. Diese erhält die Auswerteeinheit 26 ebenfalls von der Datenbank 28, wie dies durch einen Pfeil 36 schematisch angedeutet ist.

Hat die Auswerteeinheit 26 ein Auswerteergebnis ermittelt, wird dieses an eine Anzeigeeinheit 38 weitergegeben. Dies kann über einen separaten Anschluss 40 der Datenverarbeitungsanlage 14 geschehen und ist im Übrigen schematisch durch Pfeile 42 und 42' angedeutet. Für eine solche Anzeigeeinheit 38 kommen unterschiedliche Ausgestaltungsmöglichkeiten in Betracht. So ist es zum einen denkbar, spezielle Anzeigeeinheiten für den vorgesehen Zweck zu konstruieren und in die Datenverarbeitungsanlage 14 zu integrieren. Im Übrigen ist es aber auch denkbar, einen gewöhnlichen Computermonitor hierzu zu verwenden, der über den Anschluss 40 ansteuerbar ist. Neben diesen Möglichkeiten der visuellen Darstellung des Auswerteergebnisses ist es ferner denkbar, dass dieses akustisch geschieht. So kann je nachdem, wie detailliert das Auswerteergebnis einem Benutzer mitgeteilt werden soll, eine Ansage mit den notwendigen Informationen erfolgen oder auch einfach eine Mitteilung durch ein Signal stattfinden. Letzteres kann z.B. dann entweder für die positive Erkennung eines Instruments oder das Auflegen eines falschen Instruments unterschiedlich ausgestaltet sein.

Neben oder zusätzlich zu der beschriebenen Möglichkeit der Ausgabe der Auswertedaten auf der Anzeigeeinheit 38 ist es auch denkbar, dass die Datenverarbeitungsanlage 14 ferner eine Steuereinheit 44 aufweist. Diese ist in der Fig. 2 mit gestrichelten Linien als optionales Element dargestellt. Die Steuereinheit 44 kann Daten von der Auswerteeinheit 26 erhalten, wie dies schematisch durch einen Pfeil 46 angedeutet ist, und diese dann dazu nutzen, externe Geräte oder Vorrichtungen direkt oder mittelbar anzusteuern. Dies ist durch einen Pfeil 48 ebenfalls schematisch angedeutet. Beispiele für solche Vorrichtungen sind Roboter, die hier aber nicht näher gezeigt sind.

Um ein Auslösen der Bilderfassung der ersten Kamera 18 zu erreichen, ist in der Vorrichtung 10 ferner ein Taster 50 vorgesehen. Dieser Taster 50 ist funktional mit der ersten Kamera 18 verbunden. Dies ist schematisch mit Hilfe eines Pfeils 52 angedeutet. Um im Übrigen eine weitere Kommunikation zwischen einem Benutzer und der Vorrichtung 10 zu ermöglichen, weist diese ferner eine Tastatur 54 auf, die mit der Datenverarbeitungsanlage 14 verbunden ist. Dies ist schematisch mit einer Verbindungslinie 56 angedeutet.

Ein zu erkennendes medizinisches Instrument ist in der Fig. 1 schematisch als Schere 58 dargestellt. Diese Schere 58 ist auf der ersten Auflage 16 angeordnet. Damit befindet sich die Schere 58 unterhalb der ersten Kamera 18. Entsprechend der perspektivischen Ausrichtung dieser ersten Kamera 18 wird diese Schere 58 bei einer Erfassung der Bilddaten durch die erste Kamera 18 erfasst.

Um die Bildaufnahmen bzw. die Bilderfassung zu optimieren, weist die Vorrichtung 10 ferner Beleuchtungseinrichtungen auf. Diese Beleuchtungseinrichtungen sind hier schematisch durch Lampen 60 und 61 dargestellt. Um ferner Reflektionen an den zu erkennenden Instrumenten zu vermeiden, sind ferner Elemente 62 und 63 für diffuse Lichtverhältnisse vorgesehen. Diese Elemente 62 und 63 können z.B. aus Spezialfotokartonpapier hergestellt sein.

Die Funktionsweise der Feinerkennungseinheit 12 soll nun exemplarisch anhand der Vorrichtung 10 und der Fig. 1 und 2 erläutert werden. In diesem Zusammenhang wird auch das erfindungsgemäße Verfahren beschrieben.

Anhand einer vorgegebenen Packliste für ein entsprechendes Instrumentenset legt ein Benutzer die zugehörigen Instrumente auf die erste Auflage 16. Diese sind hier exemplarisch anhand der Schere 58 und einer Klemme 64 dargestellt. Die Packliste kann entweder separat für den Benutzer auf einem Ausdruck vorliegen oder mit einer separaten Anzeige dargestellt oder auch über die Anzeigeeinheit 38 dem Benutzer gezeigt werden.

Nach erfolgtem Auflegen aller Instrumente des Instrumentensets auf die erste Auflage 16 werden die Bilddaten dieses Instrumentensets auf der ersten Auflage 16 mit der ersten Kamera 18 erfasst. Dies kann in einer bevorzugten Ausführungsform durch Betätigen des Tasters 50 durch den Benutzer ausgelöst werden. Alternativ ist auch eine Auslösung durch den Benutzer mit Hilfe der Tastatur 54 denkbar.

Die durch die erste Kamera 18 erfassten Bilddaten werden nun über die erste Schnittstelle 24 an die Auswerteeinheit 26 der Datenverarbeitungsanlage 14 weitergegeben. Dort werden diese dann in der Auswerteeinheit 26 dahingehend verarbeitet, dass mit Hilfe von speziell abgestimmten Objekterkennungsalgorithmen, wie z.B. Korrelationsmethoden und Methoden der kantenbasierten Objekterkennung (z.B. generalisierter Hough-Transformation), die einzelnen Objekte, d.h. in diesem Fall Instrumente, anhand der Bilddaten erkannt werden.

Nach Erkennen dieser Instrumente bzw. Objekte werden diese dann mit Referenzbildern der entsprechenden Instrumente verglichen. Diese Referenzen sind in der Datenbank 28 hinterlegt. Da die zu packenden Instrumente für ein Instrumentenset bekannt sind, kann sich ein Vergleich darauf beschränken, dass die erkannten Objekte mit den Referenzbildern der Instrumente verglichen werden, die auch zu dem Instrumentenset gehören. Um einen solchen Vergleich durchführen zu können, werden die entsprechenden Referenzbilder aus der Datenbank 28 an die Auswerteeinheit 26 übermittelt.

Ist ein Instrument erkannt worden, wird es dann aus den vorhandenen Bilddaten entweder entfernt oder als erkannt markiert und entsprechend mit den noch übrig gebliebenen erkannten Objekten weiterverfahren. Bleiben nach dem Vergleichen aller erkannten Objekte mit den Referenzbildern der zu packenden Instrumente noch unzugeordnete Objekte in den Bilddaten übrig, gibt dies die Datenverarbeitungsanlage 14 über die Anzeigeeinheit 38 als Packfehler aus. Dies ist auch dann der Fall, wenn eine zu hohe Anzahl einer Instrumentenart auf der ersten Auflage 16 liegt.

Fehlen noch Instrumente, die auf der Packliste für ein entsprechendes Instrumentenset vorgesehen sind, so wird dies ebenfalls als Fehler in der Packsequenz auf der Anzeige 38 ausgegeben.

Ist demgegenüber das vorgesehene Instrumentenset vollständig und in der richtigen Anzahl auf der ersten Auflage 16, so wird dies als komplettes Instrumentenset ebenfalls auf der Anzeige 38 angezeigt. Der Benutzer kann dann mit dem auf der ersten Auflage 16 angeordneten Instrumentenset weiter verfahren, z.B. dieses sterilisieren.

In diesem Zusammenhang sei erwähnt, dass es für die korrekte Feinerkennung der Objekte auf der ersten Auflage 16 sinnvoll ist, die Objekte so anzuordnen, dass sie einander nicht überlagern. Dies muss aber nicht zwingend so sein und kann auch in bestimmten Erkennungsalgorithmen als Möglichkeit vorgesehen werden. Dies ist bspw. bei der Beschränkung der Objekterkennung auf Schlüsselmerkmale der Instrumente möglich.

Um die Erfassung der Bilddaten möglichst optimal zu gestalten, so dass die entsprechenden Erkennungsalgorithmen hohe Treffer erzielen können, ist es ratsam für gute Beleuchtungs- und Kontrastverhältnisse zu sorgen.

Hierzu kann die erste Auflage 16 mit einem kontrastverstärkenden Hintergrund 66 ausgestaltet sein. Dies ist in einfacher Weise beispielsweise dadurch zu realisieren, dass die erste Auflage 16 einen gleichmäßigen Farbton, z.B. blau, aufweist.

Ferner sollten die Lampen 60 und 61 eine starke Beleuchtung der ersten Auflage 16 liefern und durch die Elemente 62 und 63 gleichzeitig für möglichst diffuses Licht gesorgt werden, um unerwünschte Reflektionen zu vermindern.

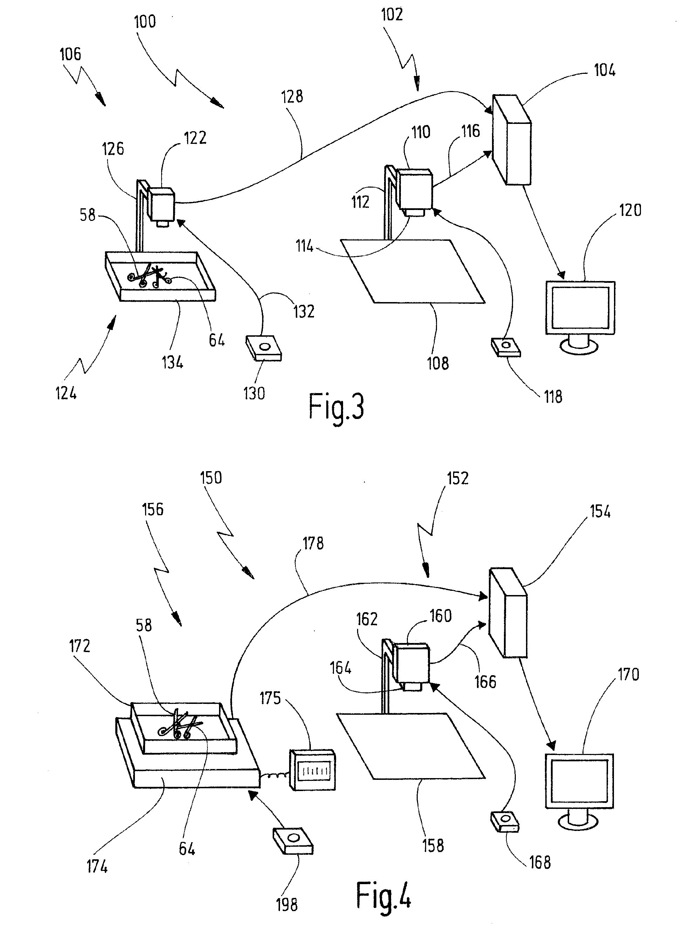

In Fig. 3 ist eine weitere erfindungsgemäße Vorrichtung 100 gezeigt.

Diese Vorrichtung 100 weist eine Feinerkennungseinheit 102, eine Datenverarbeitungsanlage 104 sowie eine Groberkennungseinheit 106 auf.

Die Datenverarbeitungsanlage 104 ist vom Aufbau ausgestaltet wie die Datenverarbeitungsanlage 14 und im Folgenden nicht näher gezeigt. Vielmehr wird auf die vorherigen Ausführungen verwiesen. Die Feinerkennungseinheit 102 weist eine erste Auflage 108 und eine erste Kamera 110 auf. Die erste Kamera 110 ist dabei an einem Stativ 112 angeordnet. Dadurch kann sie so ausgerichtet werden, dass ihr Objektiv 114 auf die erste Auflage 108 ausgerichtet ist. Die hier gezeigte erste Kamera 110 hat somit die gleiche Perspektive wie die Kamera 18 aus Fig. 1.

Die erste Kamera 110 gibt ebenfalls die erfassten Bilddaten an die Datenverarbeitungsanlage 104 weiter, wie dies durch den Pfeil 116 angedeutet ist. Die Erfassung der Bilddaten kann in gleicher Weise wie bei der Vorrichtung 10 aus Fig. 1 durch einen Taster 118 ausgelöst werden. Zur weiteren Kommunikation zwischen dem Benutzer und der Vorrichtung 100 weist die Datenverarbeitungsanlage 104 ferner eine Anzeigeeinheit 120 auf. Der übrige Aufbau und die Funktionsweise der Feinerkennungseinheit 102 zusammen mit der Datenverarbeitungsanlage 104 sind vom Prinzip identisch mit dem Aufbau und der Funktionsweise der Feinerkennungseinheit 12 zusammen mit der Datenverarbeitungsanlage 14 der Vorrichtung 10, weswegen hierzu auch auf die zuvor gemachten Erläuterungen verwiesen wird.

Zum Zwecke der besseren Ausleuchtung und der Optimierung des Kontrastverhältnisses zwischen aufgelegten Instrumenten auf der ersten Auflage 108 kann hier ebenfalls ein kontrastverstärkender Hintergrund verwendet werden, wie auch starke Beleuchtungsmittel eingesetzt werden, die hier zum Zwecke der Übersichtlichkeit nicht näher gezeigt sind.

Die zusätzliche Groberkennungseinheit 106 der Vorrichtung 100 weist eine weitere Kamera 122 auf. Diese weitere Kamera 122 ist analog zu den zuvor beschriebenen ersten Kameras 110 und 18 über einer zweiten Auflage 124 angeordnet. Hierzu ist sie an einem Stativ 126 befestigt. Die weitere Kamera 122 ist ebenfalls mit der Datenverarbeitungsanlage 104 über eine hier nicht näher gezeigte dritte Schnittstelle verbunden. Dies ist schematisch durch den Pfeil 128 angedeutet. Um auch bei der Groberkennungseinheit 106 eine Bilddatenerfassung durch den Benutzer steuern lassen zu können, weist die Groberkennungseinheit 106 ebenfalls einen Taster 130 auf. Der Taster 130 steht zur Ansteuerung funktional mit der Kamera 122 in Verbindung. Dies ist durch einen Pfeil 132 schematisch angedeutet.

Die Funktionsweise der Vorrichtung 100 ähnelt zunächst der Funktionsweise der Vorrichtung 10. Auch hier wird zunächst eine vorgegebene Packliste durch einen Benutzer abgearbeitet und entsprechende Instrumente auf die erste Auflage 108 aufgelegt und entsprechend der zuvor gemachten Ausführungen im Zusammenhang mit der Feinerkennungseinheit 12 und der Datenverarbeitungsanlage 14 durch die Feinerkennungseinheit 102 und Datenverarbeitungsanlage 104 auf Vollständigkeit und Korrektheit überprüft. Liegt die Vollständigkeit vor, wird diese ebenfalls auf der Anzeigeeinheit 120 ausgegeben, woraufhin der Benutzer dann die Instrumente auf die zweite Auflage 124 überführt. Der daraus folgende Zustand ist in der Fig. 3 schematisch wieder durch die Instrumente der Fig. 1, nämlich die Schere 58 und die Klemme 64 angedeutet.

Liegen nun alle Instrumente auf der zweiten Auflage 124, kann die weitere Kamera 122 mit der Bilderfassung beginnen. Dies kann vorzugsweise mit Hilfe des Tasters 130 durch den Benutzer ausgelöst werden. Die so ermittelten Bilddaten werden dann wie dies durch den Pfeil 128 dargestellt ist, an die Datenverarbeitungsanlage 104 weitergegeben, die dann über ihre Auswerteeinheit mit der Auswertung beginnen kann.

In einer bevorzugten Ausführungsform weist die zweite Auflage 124 ein Instrumentensieb 134 auf. In dieses Instrumentensieb 134 können dann die Instrumente des Instrumentensets, also hier die Schere 58 und Klemme 64 direkt gelegt werden, wenn sie von der Feinerkennungseinheit 102 überführt werden. Dies hat den Vorteil, dass nach erfolgreicher Groberkennung das Instrumentensieb 134 aus der Groberkennungseinheit 106 entnommen und so direkt zur Reinigung bzw. Sterilisation überbracht werden kann.

Da der zur Verfügung stehende Platz in einem solchen Instrumentensieb 134 in der Regel geringer ist als er z.B. auf der ersten Auflage 108 bzw. 16 vorliegt, kommt es in der Groberkennungseinheit häufig zur Überlagerung der zu erkennenden Instrumente, wie dies auch in der Fig. 3 für die Schere 58 und Klemme 64 angedeutet ist.

Um dennoch eine effiziente Möglichkeit zur Erkennung der Vollständigkeit des Instrumentensets zu liefern, wird hierbei vorzugsweise lediglich die Erkennung von Schlüsselmerkmalen in den Bilddaten durchgeführt. Diese Schlüsselmerkmale können beispielsweise vorher durch das System erlernt oder direkt vom Benutzer vorgegeben worden sein.

Ein weiterer Vorteil der Verwendung von Schlüsselmerkmalen zur Erkennung der Instrumente ist, dass der nachfolgende Groberkennungsschritt durch die Groberkennungseinheit 106 vergleichsweise wenig Zeit beansprucht.

Durch die Verwendung der Groberkennungseinheit 106 hat die Gesamtvorrichtung 100 den Vorteil, dass das so bestückte Instrumentensieb 134 dahingehend abschließend geprüft wird, ob das Instrumentenset vollständig vorliegt und das Instrumentensieb 134 somit korrekt bestückt wurde. Fehler dergestalt, dass ein Instrument von einer vorherigen Erkennung, beispielsweise durch die Feinerkennungseinheit 102 nicht korrekt in das zugehörige Instrumentensieb 134 überführt wird, werden dadurch ausgeschlossen.

Neben der zuvor beschriebenen und in den folgenden Ausführungen dargestellten Möglichkeit einer separaten Groberkennungseinheit neben einer Feinerkennungseinheit, ist es im Rahmen dieser Erfindung ebenfalls denkbar, die Einheiten konstruktiv zusammenzulegen. Damit wären erste und zweite Auflage sowie erste und weitere (oder zweite und weitere) Kamera identisch. Eine solche Anordnung hat einen geringeren Platzbedarf gegenüber einer separaten Anordnung.

Fig. 4 zeigt eine weitere erfindungsgemäße Vorrichtung 150.

Diese erfindungsgemäße Vorrichtung 150 weist ebenfalls eine Feinerkennungseinheit 152, eine Datenverarbeitungseinheit 154 und eine Groberkennungseinheit 156 auf.

Die Feinerkennungseinheit 152 weist in vergleichbarer Weise zu den Vorrichtungen 10 und 100 eine erste Auflage 158 sowie eine erste Kamera 160 mit Stativ 162 und Objektiv 164 auf. Auch die erste Kamera 160 steht in Verbindung mit der Datenverarbeitungsanlage 154, wie dies durch einen Pfeil 166 angedeutet ist. Auch die erste Kamera 160 kann durch einen Taster 168 ausgelöst werden.

Die Funktionsweise der Feinerkennungseinheit 152 ist analog zu der Feinerkennungseinheit 102, weswegen hier auf weitere Ausführungen verzichtet und lediglich auf die vorherigen Ausführungen verwiesen wird. Ein entsprechendes Ergebnis der Identifizierung der Instrumente erfolgt dann mit Bezug auf das zu bestückende Instrumentenset über eine Anzeigeeinheit 170.

Ebenfalls analog zu der Vorrichtung 100 aus Fig. 3 soll anschließend an die Überprüfung der Vollständigkeit eines entsprechenden Instrumentensets eine Groberkennung in der Groberkennungseinheit 156 vorgenommen werden.

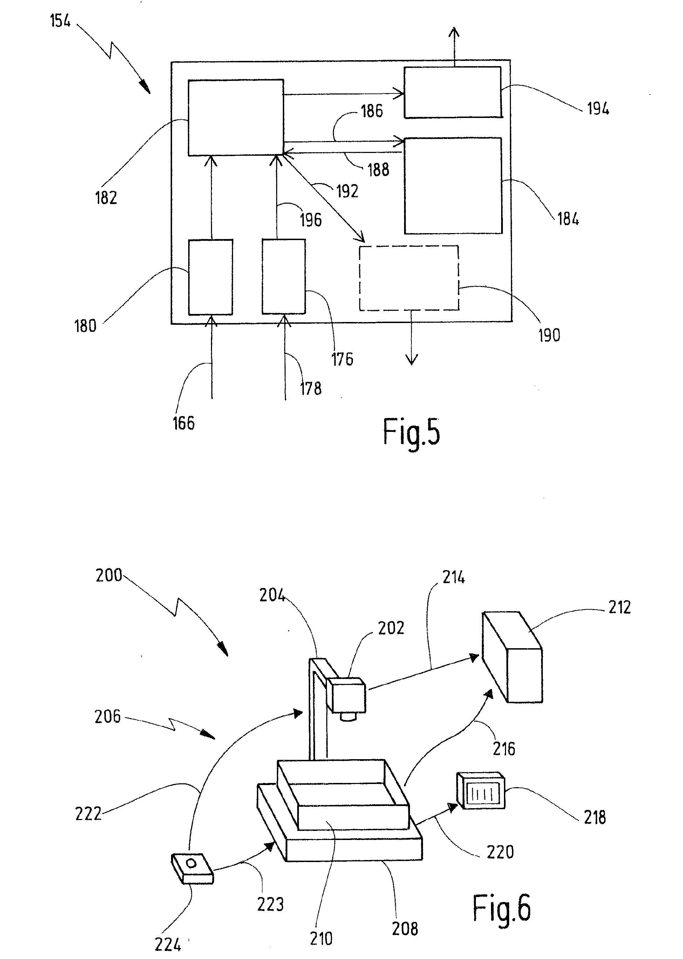

Hierzu weist die Groberkennungseinheit 156 auch in der bevorzugten Ausführungsform ein Instrumentensieb 172 auf. Dieses Instrumentensieb 172 ist auf einer Waage 174 angeordnet. Die Waage 174 weist zum einen eine eigene Anzeigeeinheit 175 auf. Ferner steht die Waage 174 mit der Datenverarbeitungsanlage 154 über eine zweite Schnittstelle 176 in Verbindung. Dies ist durch einen Pfeil 178 angedeutet und auch in Fig. 5 zu erkennen.

Die in Fig. 5 schematisch gezeigte Datenverarbeitungsanlage 154 stimmt im Wesentlichen mit der Datenverarbeitungsanlage 14 der Fig. 2 überein. Auch hierbei gibt es eine erste Schnittstelle 180 zur Aufnahme der Bilddaten aus der ersten Kamera 160. Dies ist schematisch durch einen Pfeil 166 angedeutet. Weiterhin weist die Datenverarbeitungsanlage 154 auch eine Auswerteeinheit 182 sowie eine Datenbank 184 auf. Die Auswerteeinheit 182 kann Daten in die Datenbank 184 schreiben und aus dieser auslesen, wie dies durch Pfeile 186 und 188 angedeutet ist. Im Übrigen weist die Datenverarbeitungsanlage 154 auch eine optionale Steuereinheit 190 auf, die ebenfalls von der Auswerteeinheit 182 mit Daten zur Steuerung weiterer Geräte und Vorrichtungen versorgt werden kann. Dies ist durch einen Pfeil 192 angedeutet. Auch weist die Datenverarbeitungsanlage 154 einen Anschluss 194 auf, über den eine nicht näher gezeigte Verbindung zu der Anzeigeeinheit 170 erfolgen kann.

Als Unterschied zur Datenverarbeitungsanlage 14 weist die Datenverarbeitungsanlage 154 zusätzlich die zweite Schnittstelle 176 auf. Diese dient, wie zuvor bereits beschrieben, zur Weitergabe der Daten der Waage 174 an die Auswerteeinheit 182. Dies ist schematisch durch einen Pfeil 196 angedeutet.

Werden nun wie dies in Fig. 4 angedeutet ist, wieder Instrumente, hier die Schere 58 und die Klemme 64, als fertiges Instrumentenset in das Instrumentensieb 172 gelegt, bestimmt die Waage das Gewicht dieses Instrumentensets. Dies geschieht vorzugsweise nach Betätigen eines Tasters 198. Mit Gewicht des Instrumentensets ist bevorzugt dessen Gesamtgewicht gemeint. Dies geschieht vorzugsweise nach vorheriger Tarierung der Waage mit dem Instrumentensieb 172, um Ungleichmäßigkeiten in den Gewichten der verwendeten Instrumentensiebe zu kompensieren.

Die so erhaltenen Gewichtsdaten werden dann über die zweite Schnittstelle 176 zur Auswerteeinheit 182 der Datenverarbeitungsanlage 154 weitergegeben. Dort erfolgt ein Vergleich mit dem Sollgewicht des vorliegenden Instrumentensets aus Schere 58 und Klemme 64. Dieses ist als Referenz in der Datenbank 184 hinterlegt. Stimmt das ermittelte Gewicht von Schere 58 und Klemme 64 mit dem hinterlegten Sollgewicht überein, wird von der Datenverarbeitungsanlage 154 eine entsprechende Mitteilung an den Benutzer über die Anzeige 170 gegeben.

Weicht das ermittelte Gewicht jedoch von dem hinterlegten Sollgewicht nach Berücksichtigung eventueller Toleranzen ab, wird dies als Fehler im Instrumentenset an den Benutzer über die Anzeigeeinheit 170 mitgeteilt.

Hieraufhin hat der Benutzer das vermeintlich vollständige Instrumentenset noch einmal zu überprüfen. Mögliche Fehler können hierbei falsche Übertragung der Instrumente von Feinerkennungseinheit zu Groberkennungseinheit sein, als auch Fehler insbesondere beim Zusammenbau von komplexeren Instrumenten. So können z.B. einzelne innen liegende Bauteile fehlen. Dies ist nicht von außen zu erkennen und somit nicht mittels der vorherigen Feinerkennung in der Feinerkennungseinheit 152 zu ermitteln.

Die Hinterlegung des Referenzgewichts bzw. Sollgewichts eines jeweiligen Instrumentensets in der Datenbank 184 kann unterschiedlich geschehen. Eine Möglichkeit ist hierbei die Hinterlegung der Einzelgewichte der jeweiligen Instrumente. Diese würden dann von der Datenverarbeitungsanlage 154 für jedes entsprechende Instrumentenset jeweils neu berechnet und dann entsprechend mit den ermittelten Gewichten verglichen werden. Alternativ kann auch eine Bestimmung von Sollgewichten kompletter Instrumentensets erfolgen und somit für jedes Instrumentenset als fester Wert in der Datenbank 184 abgelegt werden.

Die Bestimmung der Soll- bzw. Referenzgewichte der Instrumente oder Instrumentensets geschieht entweder durch die Aufnahme von Herstellerdaten in die Datenverarbeitungsanlage 154 oder durch eigene Wägungen in einer Lernphase. Hierbei werden vorzugsweise mehrere Wägevorgänge mit den jeweiligen Instrumenten(sets) vorgenommen, so dass, insbesondere bei unterschiedlichen Umgebungsbedingungen wie Feuchtigkeit und Temperatur, mehrere Gewichte für ein Instrument bzw. Instrumentenset vorliegen. Dadurch kann dann eine entsprechende Fehlertoleranz anhand der Standardabweichung ermittelt und hinterlegt werden.

Neben der zuvor gezeigten Ausgestaltung der Vorrichtung 100 und 150 mit den Groberkennungseinheiten 106 und 156 ist in Fig. 6 eine weitere bevorzugte Ausführungsform in Form einer Groberkennungseinheit 200 gezeigt.

Wenngleich die Groberkennungseinheit 200 hier als einzelnes Element dargestellt ist, so versteht es sich, dass diese in beliebiger Form mit den zuvor gezeigten Feinerkennungseinheiten 12, 102 und 152 kombiniert werden kann.

Die Groberkennungseinheit 200 kann als eine Kombination der Groberkennungseinheiten 106 und 156 angesehen werden.

Die Groberkennungseinheit 200 weist eine weitere Kamera 202 auf, die an einem Stativ 204 angeordnet ist. Die Anordnung der weiteren Kamera 202 ist in analoger Weise so, dass sie über einer zweiten Auflage 206 angeordnet und so ausgerichtet ist, dass die Kameraperspektive auf diese zweite Auflage 206 gerichtet ist. Auch die zweite Auflage 206 weist eine Waage 208 auf. Auf dieser Waage 208 ist ein Instrumentensieb 210 angeordnet. Sowohl die weitere Kamera 202 als auch die Waage 208 sind mit einer Datenverarbeitungsanlage 212 verbunden, wie dies schematisch durch Pfeile 214 und 216 angedeutet ist.

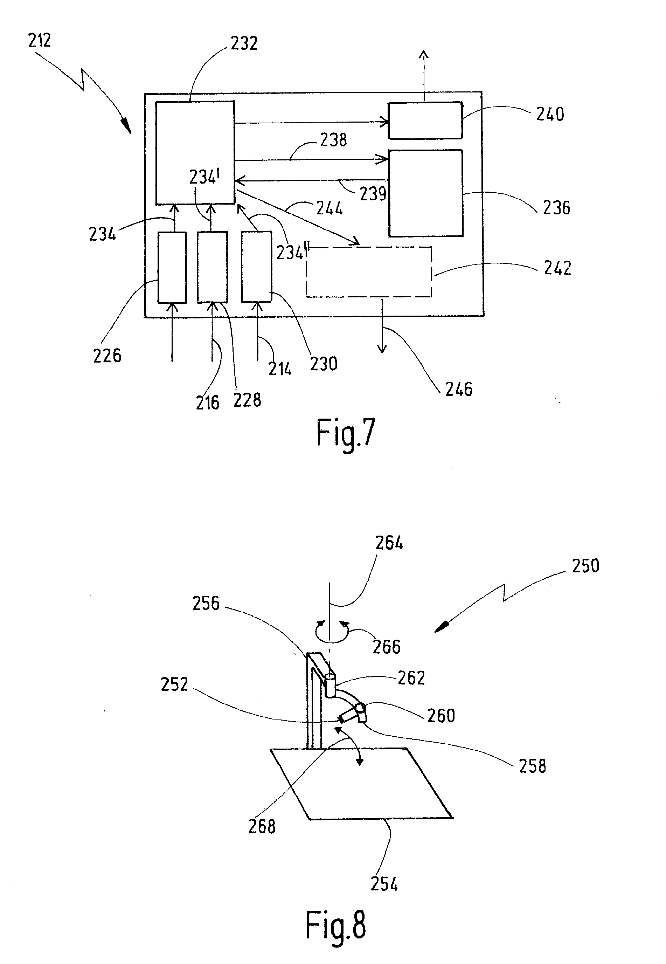

Die Datenverarbeitungsanlage 212 ist im Übrigen analog zu den zuvor beschriebenen Datenverarbeitungsanlagen 14, 104 und 154 ausgestaltet und näher im Zusammenhang mit der Fig. 7 beschrieben. Sie steht im Übrigen in Verbindung mit einer Anzeigeeinheit, die der Übersichtlichkeit halber nicht in der Fig. 6 dargestellt ist.

Zusätzlich kann die Waage noch mit einer eigenen Anzeigeeinheit 218 verbunden sein, wie dies schematisch durch einen Pfeil 220 angedeutet ist. Die Anzeigeeinheit 218 dient dann dazu, das jeweilige Gewicht der auf der Waage 208 angeordneten Gegenstände anzuzeigen.

Um den Bilderfassungs- und Wägevorgang auszulösen, weisen auch hier die weitere Kamera 202 und Waage 208 eine Ansteuerung durch Taster auf, wie dies durch Pfeile 222 und 223 schematisch angedeutet ist. Dies kann durch separate einzelne Taster geschehen oder, wie hier in Fig. 6 dargestellt, durch einen gemeinsamen Taster 224 erfolgen.

Der Ablauf der Groberkennung mit der Groberkennungseinheit 200 läuft im Wesentlichen analog zu den zuvor beschriebenen Groberkennungseinheiten 106 und 156 ab. Unterschied zu der zuvor beschriebenen Groberkennung ist hierbei jedoch die Möglichkeit zur simultanen Erkennung mittels der weiteren Kamera 202 und der Waage 208.

Diese Variante hat den Vorteil, dass zum einen Fehler beim Zusammenbau von komplexeren Instrumenten aufgrund einer Gewichtsabweichung detektiert werden können, während trotzdem die optischen individuellen Schlüsselmerkmale der jeweiligen Instrumente nochmals überprüfbar sind. Dadurch kann sichergestellt werden, dass wirklich jedes der vorgesehenen Instrumente des Instrumentensets im Instrumentensieb 210 angeordnet wurde und korrekt zusammengebaut ist.

Um diese Daten aufnehmen zu können, weist die Datenverarbeitungsanlage 212 neben einer ersten Schnittstelle 226 nun eine zweite Schnittstelle 228 und dritte Schnittstelle 230 auf. Dies ist insbesondere in Fig. 7 zu sehen. Alle Schnittstellen 226, 228 und 230 geben ihre Daten an eine Auswerteeinheit 232 weiter, wie dies durch Pfeile 234, 234' und 234" angedeutet ist. Die Auswerteeinheit 232 kann nun die so erhaltenen Daten mit einer Datenbank 236 austauschen bzw. mit den Referenzdaten aus dieser Datenbank 236 vergleichen. Dies ist schematisch durch Pfeile 238 und 239 angedeutet. Somit können in der Auswerteeinheit 232 neben den Bilddaten aus den jeweiligen Feinerkennungseinheiten auch die Bilddaten der Groberkennungseinheit 200 sowie die Wägedaten der Groberkennungseinheit 200 mit entsprechenden Referenzdaten verglichen werden.

Im Übrigen weist die Datenverarbeitungsanlage 212 auch einen Anschluss 240 zur Ausgabe der Daten an eine nicht näher gezeigte Anzeigeeinheit auf. Auch kann bei der Datenverarbeitungsanlage 212 wieder optional eine Steuereinheit 242 vorgesehen sein, um entsprechende Vorrichtungen oder Einheiten aufgrund der ausgewerteten Daten zu steuern. Hierbei ist die Übertragung der Daten von der Auswerteeinheit 232 auf die Steuereinheit 242 durch einen Pfeil 244 angedeutet. Die Ansteuerung von externen Einheiten und Vorrichtungen deutet ein Pfeil 246 an.

Fig. 8 zeigt eine Kameraanordnung 250. Diese Kameraanordnung 250 kann mit ihrer Ausgestaltung auf die jeweiligen Feinerkennungseinheiten 12, 102, 152 oder Groberkennungseinheiten 106 und 200 entsprechend übertragen werden.

Die Kameraanordnung 250 weist eine Kamera 252 auf, die ebenfalls oberhalb einer Auflage 254 angeordnet ist. Die Anordnung der Kamera 252 oberhalb der Auflage 254 erfolgt mit Hilfe eines Stativs 256. Die Kameraanordnung 250 ist hierbei so, dass die Kamera 252 in ihrer Position veränderbar angeordnet ist und dabei eine halbkugelförmige Fläche an eigenen Positionen abdecken kann. Dabei ist die Kamera 252 so angeordnet, dass sie stets mit ihrer Kameraperspektive in Richtung auf die Auflage 254 ausgerichtet ist.

In dem vorliegenden Beispiel wird dies dadurch erreicht, dass das Stativ 256 einen Bogen 258 aufweist. An diesem Bogen 258 ist die Kamera 252 beweglich entlang dieses Bogens 258 angeordnet. Diese Bewegung und Anordnung kann beispielsweise durch einen hier schematisch dargestellten Motor 260 erfolgen. Der Bogen 258 ist seinerseits an einem Ende über einen zweiten Motor 262 an dem Stativ 256 angeordnet.

Der Bogen 258 kann somit in der Folge Rotationen um eine Achse 264 des Motors 262 durchführen, wie dies durch einen Doppelpfeil 266 angedeutet ist. Ebenso kann die Kamera 252 durch den Motor 260 entlang des Bogens 258 bewegt werden, wie dies durch einen Doppelpfeil 268 angedeutet ist.

Letztendlich gestattet es diese Ausgestaltung einer Kameraanordnung 250 für die jeweiligen Fein- und Groberkennungseinheiten Bilddaten bzw. Aufnahmen aus zwei oder beliebig vielen unterschiedlichen Perspektiven vornehmen zu können.

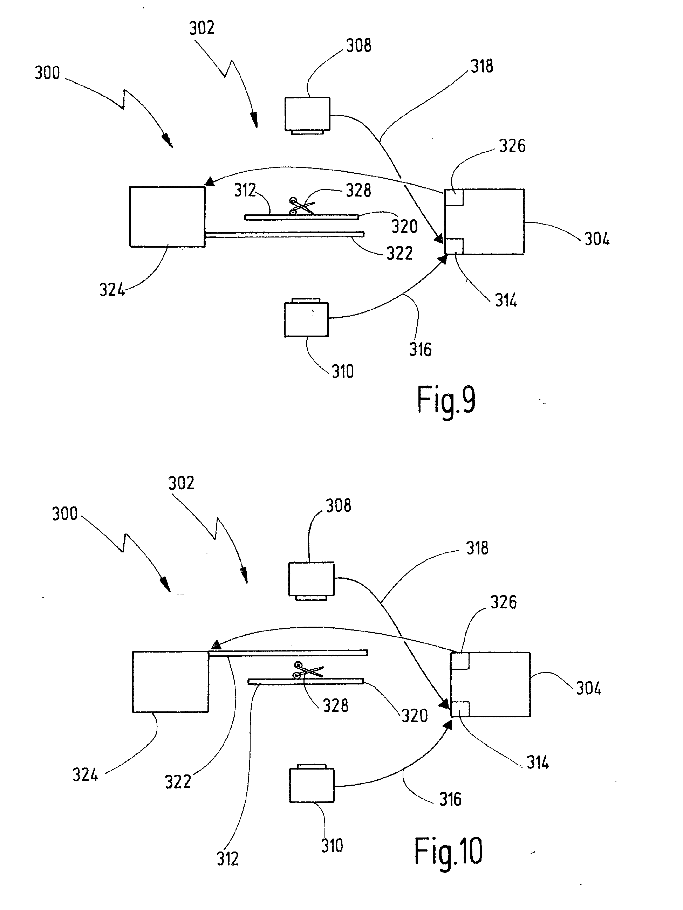

Fig. 9 und 10 zeigen eine weitere erfindungsgemäße Vorrichtung 300. Die Vorrichtung 300 weist eine Feinerkennungseinheit 302, eine Datenverarbeitungsanlage 304 und eine Groberkennungseinheit auf, wobei die Letztere zum Zwecke der Übersichtlichkeit nicht näher dargestellt ist. Diese Groberkennungseinheit ist so ausgestaltet, wie eine der zuvor bereits gezeigten und erläuterten Groberkennungseinheiten 106, 156 oder 200.

Neben einer ersten Kamera 308 weist die Feinerkennungseinheit 302 zusätzlich eine zweite Kamera 310 auf. Die erste Kamera 308 und zweite Kamera 310 sind so zueinander angeordnet, dass sie jeweils auf einer Seite einer ersten Auflage 312 der Feinerkennungseinheit 302 angeordnet sind.

Beide Kameras 308 und 310 stehen mit der Datenverarbeitungsanlage 304 über eine hier schematisch angedeutete erste Schnittstelle 314 in Verbindung. Dies ist schematisch durch Pfeile 316 und 318 angedeutet.

Die erste Auflage 312 weist im vorliegenden Ausführungsbeispiel eine transparente Grundfläche 320 sowie einen kontrastverstärkenden Hintergrund 322 auf.

Die transparente Grundfläche 320 kann dabei aus beliebigen transparenten Materialien gefertigt sein. Diese müssen es lediglich gestatten, entsprechende Bildaufnahmen zu ermöglichen und Instrumente auf diese Grundfläche 320 aufzulegen. Als Beispiele seien hierfür Glas oder transparente Kunststoffe, wie z.B. Plexiglas genannt.

Der kontrastverstärkende Hintergrund 322 ist hier als eine bewegliche einfarbige Platte ausgebildet. Ferner ist der kontrastverstärkende Hintergrund 322 an einer Bewegungseinheit 324 angeordnet. Diese Bewegungseinheit 324 vermag den kontrastverstärkenden Hintergrund 322 von einer Position unterhalb der transparenten Grundfläche 320 in eine Position oberhalb der transparenten Grundfläche 320 zu übertragen - jeweils in Bezug auf die Darstellung der Fig. 9 und 10. Dies kann beispielsweise durch Rotation oder durch eine Bewegungsabfolge der Art seitliches Verschieben, Anheben und wieder Zurückbewegen erfolgen. Ebenfalls denkbar ist eine hier nicht gezeigte Anordnung auf Rollbändern, die neben der transparenten Grundfläche 320 verlaufen und es gestatten, den kontrastverstärkenden Hintergrund 322 zum einen seitlich neben die transparente Grundfläche 320 zu verschieben und vertikal in der Höhe zu verstellen, um ihn dann anschließend wieder über bzw. unter die transparente Grundfläche 320 zurückzuschieben.

Die Bewegungseinheit 324 steht im Übrigen mit einer hier ebenfalls nur schematisch angedeuteten Steuereinheit 326 der Datenverarbeitungsanlage 304 in Verbindung.

Diese Steuereinheit 326 erhält, wie dies bereits in den zuvor beschriebenen Ausführungsbeispielen der Datenverarbeitungsanlagen als Option erläutert wurde, Informationen und Signale von einer hier nicht näher gezeigten Auswerteeinheit der Datenverarbeitungsanlage 304 und regelt somit in diesem Fall das Verschieben und Verstellen des kontrastverstärkenden Hintergrunds 322 über die Bewegungseinheit 324.

Zum Zwecke einer kompletten Automatisierung wäre es ferner denkbar, dass die Steuereinheit 326 hier auch das Auslösen der ersten Kamera 308 und zweiten Kamera 310 und die damit verbundene Erfassung der Bilddaten steuert.

Hinsichtlich des Vorgangs der Erfassung der Bilddaten eines entsprechend hier schematisch angedeuteten Instruments, eine Schere 328, wird zunächst durch die erste Kamera 308 eine Bilderfassung der Schere 328 vorgenommen. Der kontrastverstärkenden Hintergrund 322 ist dabei so unterhalb der transparenten Grundfläche 320 angeordnet, dass dieser weiterhin die Objekterkennungseigenschaften positiv beeinflusst, indem er den Kontrast zwischen dem zu erkennenden Objekt, hier der Schere 328, und dem Hintergrund erhöht.

Anschließend wird nach Eingang der Bilddaten bei der Datenverarbeitungsanlage 308 mittels der Steuereinheit 328 über die Bewegungseinheit 324 der kontrastverstärkende Hintergrund 322 in seiner Position verschoben. Dabei wird er von unterhalb der transparenten Grundfläche 320 in eine Position oberhalb der transparenten Grundfläche 320 überbracht. Dies ist anhand des Übergangs von Fig. 9 zu Fig. 10 nachvollziehbar.

Nach erfolgter Positionierung des kontrastverstärkenden Hintergrunds 322 kann nun die zweite Kamera 310 mit der Erfassung der Bilddaten beginnen. Hierbei kann diese durch die transparente Grundfläche 320 hindurch die Bilddaten der mit Bezug auf die Darstellung von Fig. 10 unteren Seite der Schere 328 erfassen. Auch hier sorgt wieder der kontrastverstärkenden Hintergrund 322 für einen ausreichenden Unterschied zwischen zu erkennendem Objekt und Hintergrund, um eine Objekterkennung zu begünstigen.

Wenngleich die Beschreibung dieses Verfahrens in dieser Reihenfolge vorgenommen wurde, ist es auch möglich, die Reihenfolge umzukehren, d.h. zunächst die Unterseite und dann die Oberseite einer Schere 328 in Form von Bilddaten zu erfassen.

Die so erhaltenen Bilddaten von erster Kamera 308 und zweiter Kamera 310 werden sodann an die Datenverarbeitungsanlage 304 übermittelt und entsprechend den zuvor gemachten Ausführungen in der hier nicht näher gezeigten Auswerteeinheit der Datenverarbeitungsanlage 304 ausgewertet. Dadurch wird das Objekt, hier die Schere 328 identifiziert und hinsichtlich ihrer Zugehörigkeit zu der entsprechenden Packsequenz des zu packenden Instrumentensets untersucht.

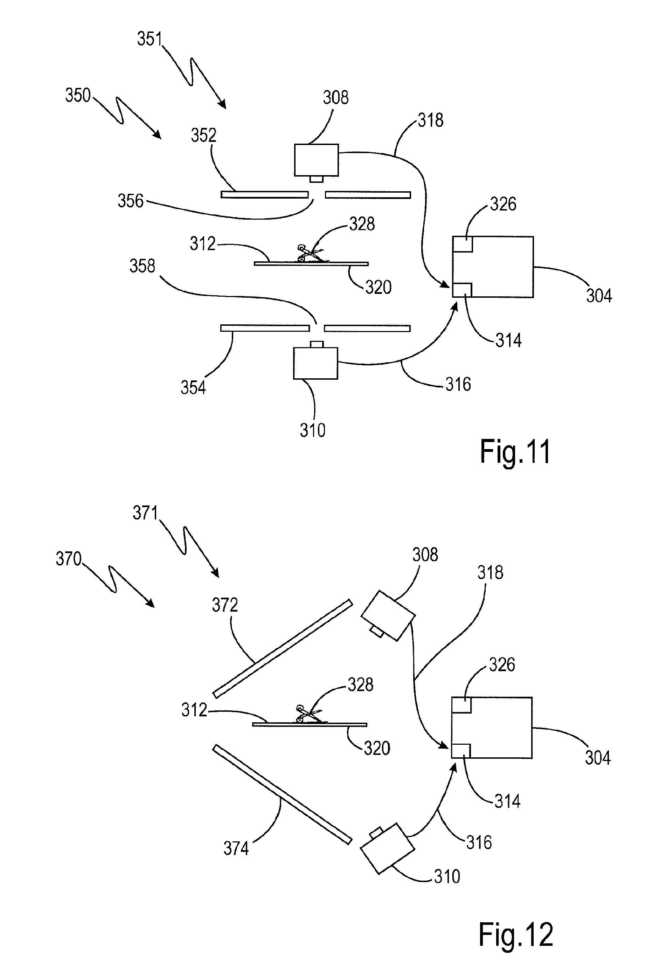

Die in Fig. 11 und 12 dargestellten Vorrichtungen 350 und 370 weisen teilweise gleiche Komponenten wie die Vorrichtung 300 aus Fig. 9 und 10 auf. Entsprechend sind gleiche Merkmale mit den gleichen Bezugszeichen versehen und werden im Folgenden nicht näher im Detail erläutert.

Im Unterschied zur Vorrichtung 300 aus Fig. 9 und 10 weist die Vorrichtung 350 aus Fig. 11 eine Feinerkennungseinheit 351 mit zwei kontrastverstärkenden Hintergründen 352 und 354 auf. Diese sind auf jeweils einer Seite der ersten Auflage 312 angeordnet. Dabei befinden sich diese kontrastverstärkenden Hintergründe 352 und 354 jeweils zwischen der ersten Auflage 312 und der jeweiligen Kamera 308 bzw. 310. Um dennoch eine Aufnahme der Instrumente auf der ersten Auflage 312, bzw. der transparenten Grundfläche 320 zu ermöglichen, weisen die kontrasverstärkenden Hintergründe 352 und 354 jeweils eine Öffnung 356 bzw. 358 auf. Durch diese Öffnungen 356 und 358 können die Kameras 308 und 310 jeweils Aufnahmen von einem Instrument, z.B. der Schere 328, auf der transparenten Grundfläche 320 machen. Dabei dient dann jeweils der gegenüberliegende kontrastverstärkende Hintergrund 352 bzw. 354 zur Verbesserung der Bildeigenschaften für die Objekterkennung, wie dies bereits zuvor erläutert wurde.

Mit Bezug auf die Darstellung in Fig. 11 dient der kontrastverstärkende Hintergrund 352 als Hintergrund für die Aufnahme mit Kamera 310 und der kontrastverstärkende Hintergrund 354 als Hintergrund für die Aufnahme mit Kamera 308. Dabei auftretende Aufnahmen der jeweils gegenüberliegenden Kamera 308 bzw. 310 und der entsprechenden Öffnungen 356 bzw. 358 können bei der Bildverarbeitung entsprechend berücksichtigt und aus den Bilddaten herausgerechnet werden. Dies ist beispielsweise durch die Bildung von Differenzbildern, d.h. durch Aufnahmen mit und ohne Instrumenten möglich. Mit der Vorrichtung 350 sind aufgrund der in diesem Beispiel fixen Anordnung von Kamera 308 und 310, sowie kontrastverstärkenden Hintergründen 352 und 354 sogar gleichzeitige und damit zeitsparenden Aufnahmen möglich, da ein Um- bzw. Verstellen von Kamera und/oder kontrastverstärkendem Hintergrund entfällt.

Auch die in Fig. 12 dargestellte Vorrichtung 370 weist eine Feinerkennungseinheit 371 mit zwei kontrastverstärkenden Hintergründen 372 und 374 auf. Im Gegensatz zur Vorrichtung 350 aus Fig. 11 sind diese jedoch mit Bezug zu einer durch die erste Auflage 312 bzw. die transparente Grundfläche 320 angedeutete Ebene schräg ausgerichtet. Diese Ausrichtung der kontrastverstärkenden Hintergründe 372 und 374 ist so, dass sie jeweils als Hintergrund für die ebenfalls hier mit ihrer jeweiligen Perspektive schräg zur transparenten Grundfläche 320 angeordneten Kamera 308 bzw. 310 dienen und dadurch entsprechend der zuvor gemachten Ausführungen die Objekterkennung in den Aufnahmen durch die Kameras 308 und 310 verbessern bzw. erleichtern. Die hier im Beispiel gezeigte Anordnung ermöglicht es, dass z.B. die Aufnahme der Schere 328 durch die Kamera 310 vor dem kontrastverstärkendem Hintergrund 372 erfolgt. Entsprechend dient der kontrastverstärkende Hintergrund 374 als Hintergrund für Aufnahmen mit der Kamera 308. Die Kamera 308 bzw. 310 ist hier seitlich zum kontrastverstärkenden Hintergrund 372 bzw. 374 angeordnet. Dieser kann daher ohne eine Öffnung, wie sie bei der Vorrichtung 350 in den kontrastverstärkenden Hintergründen 352 und 354 vorliegt, ausgestaltet werden. Eine Aufnahme durch diesen hindurch wird somit durch die jeweils schrägen Anordnungen vermieden. Ebenfalls entfällt ein eventuell notwendiges Bearbeiten der Bilddaten aufgrund der gleichzeitig mit aufgenommenen Kamera 308 bzw. 310 und der jeweiligen Öffnungen 356 bzw. 358.

Auch hierbei wird eine Aufnahme mit beiden Kameras 308 und 310 gleichzeitig und ohne weitere Verzögerungen durch ein Verstellen eines kontrastverstärkenden Hintergrundes und/oder einer Kamera ermöglicht. Kamera 308 und 310 sowie kontrastverstärkende Hintergründe 372 und 374 sind hierfür ebenfalls vorzugsweise fest bzw. fix angeordnet.

Es versteht sich, dass die zuvor gemachten Ausführungen zur Ausgestaltung einer Kamera, insbesondere in Fig. 8, auch auf die hier gezeigten Kameras 308 und 310 der Fig. 9, 10, 11 und 12 übertragbar sind. Ferner versteht sich auch, dass entsprechende Beleuchtungseinheiten in dieser Vorrichtung vorgesehen sind und lediglich zum Zwecke der Vereinfachung und Übersichtlichkeit der Darstellungen nicht näher gezeigt sind. Neben der zuvor dargestellten Verwendung von Lampen ist es im Übrigen auch denkbar, in den hier gezeigten Kameras 308 und 310, sowie auch in allen anderen im vorhergehenden beschriebenen Kameras und Kameravorrichtungen, Beleuchtungseinrichtungen in oder an der Kamera selber anzuordnen. Dadurch ergibt sich eine kompakte platzsparende Anordnung.

Ferner versteht sich, dass auch die einzeln dargestellten Feinerkennungseinheiten 302, 351 und 371 jeweils mit den zuvor beschriebenen Groberkennungseinheiten 106, 156 bzw. 200 kombiniert werden können. Vorzugsweise weisen die Vorrichtungen 350 und 370 ebenfalls eine Groberkennungseinheit auf, die jeweils entsprechend einer der Groberkennungseinheiten 106, 156 oder 200 ausgestaltet ist.

## Patentansprüche

1. Vorrichtung zum Zusammenstellen von medizinischen Instrumentensets, mit einer Datenverarbeitungsanlage (14, 104, 154, 212, 304) und einer Feinerkennungseinheit (12, 102, 152, 302, 351, 371), wobei die Datenverarbeitungsanlage (14, 104, 154, 212, 304) eine Anzeigeeinheit (38, 120, 170, 218), eine Datenbank (28, 184, 236), eine erste Schnittstelle (24, 180, 226) und eine Auswerteeinheit (26, 182, 232) aufweist, und wobei die Feinerkennungseinheit (12, 102, 152, 302, 351, 371) eine erste Auflage (16, 108, 158, 312) zum Auflegen der medizinischen Instrumente eines Instrumentensets und eine erste Kamera (18, 110, 160, 308) aufweist, und die erste Kamera (18, 110, 160, 308) so angeordnet und ausgerichtet ist, dass sie Bilddaten von zu erkennenden medizinischen Instrumenten (58, 64, 328) auf der ersten Auflage (16, 108, 158, 312) aus zumindest einer Perspektive erfassen kann, und wobei die Datenverarbeitungsanlage (14, 104, 154, 212, 304) so ausgestaltet ist, dass sie über die erste Schnittstelle (24, 180, 226) Bilddaten von der ersten Kamera (18, 110, 160, 308) erhält und die erhaltenen Bilddaten in der Datenbank (28, 184, 236) speichern und mittels der Auswerteeinheit (26, 182, 232) mit bereits gespeicherten Bilddaten von medizinischen Instrumenten (58, 64, 328) vergleichen und dadurch ein jeweiliges medizinisches Instrument (58, 64, 328) optisch identifizieren kann, **dadurch gekennzeichnet, dass** die Vorrichtung ferner eine Groberkennungseinheit (106, 156, 200, 306) aufweist, wobei die Groberkennungseinheit (106, 156, 200, 306) eine Auflage (124, 174, 206) aufweist, auf die die Instrumente (58, 64, 328) nach der optischen Identifizierung in der Feinerkennungseinheit (12, 102, 152, 302, 351, 371) zu einem Instrumentenset zusammengeführt werden.

2. Vorrichtung nach Anspruch 1, **dadurch gekennzeichnet, dass** die Auflage (124, 174, 206) der Groberkennungseinheit (106, 156, 200, 306) durch eine zweite Auflage (124, 174, 206) gebildet wird.

3. Vorrichtung nach Anspruch 1, **dadurch gekennzeichnet, dass** die Auflage (124, 174, 206) der Groberkennungseinheit (106, 156, 200, 306) durch die erste Auflage (16, 108, 158, 312) gebildet wird.

4. Vorrichtung nach einem der Ansprüche 1 bis 3, **dadurch gekennzeichnet, dass** die erste Auflage (16, 108, 158, 312) zumindest einen kontrastverstärkenden Hintergrund (66, 322, 352, 354, 372, 374) aufweist, der so angeordnet ist, dass er aus der jeweiligen Kameraperspektive hinter einem zu erkennenden Instrument (58, 64, 328) liegt.

5. Vorrichtung nach einem der Ansprüche 1 bis 4, **dadurch gekennzeichnet, dass** die erste Kamera (18, 110, 160, 308) in ihrer Position veränderbar angeordnet ist, so dass sie Bilddaten aus zumindest zwei Perspektiven erfassen kann.

6. Vorrichtung nach einem der Ansprüche 1 bis 5, **dadurch gekennzeichnet, dass** die erste Auflage (16, 108, 158, 312) eine transparente Grundfläche (320) aufweist, auf die die zu erkennenden Instrumente (58, 64, 328) aufgelegt werden.

7. Vorrichtung nach Anspruch 6, **dadurch gekennzeichnet, dass** die Feinerkennungseinheit (12, 102, 152, 302, 351, 371) ferner eine zweite Kamera (310) aufweist, die so angeordnet und ausgerichtet ist, dass sie Bilddaten von zu erkennenden Instrumenten (58, 64, 328) auf der ersten Auflage (16, 108, 158, 312) aus zumindest einer weiteren Perspektive erfassen kann, und wobei die Datenverarbeitungsanlage (14, 104, 154, 212, 304) so ausgestaltet ist, dass sie über eine Schnittstelle (24, 180, 226) zusätzlich Bilddaten von der zweiten Kamera (310) erhält und die erhaltenen Bilddaten in der Datenbank (28, 184, 236) speichern und mittels der Auswerteeinheit (26, 182, 232) mit bereits gespeicherten Bilddaten von Instrumenten (58, 64, 328) vergleichen und dadurch ein jeweiliges Instrument (58, 64, 328) optisch identifizieren kann, und wobei die erste Kamera (18, 110, 160, 308) auf der einen und die zweite Kamera (310) auf der anderen gegenüberliegenden Seite der Grundfläche (320) angeordnet sind.

8. Vorrichtung nach Anspruch 7, **dadurch gekennzeichnet, dass** die zweite Kamera (310) in Ihrer Position veränderbar angeordnet ist, so dass sie Bilddaten aus zumindest zwei Perspektiven erfassen kann.

9. Vorrichtung nach einem der Ansprüche 6 bis 8, **dadurch gekennzeichnet, dass** der zumindest eine kontrastverstärkende Hintergrund (66, 322) zwischen mehreren Positionen verschiebbar sein kann, wobei die mehreren Positionen jeweils auf gegenüberliegenden Seiten der transparenten Grundfläche (320) liegen.

10. Vorrichtung nach einem der Ansprüche 6 bis 8, **dadurch gekennzeichnet, dass** die Vorrichtung zumindest zwei kontrastverstärkende Hintergründe (352, 354, 372, 374) aufweist, die jeweils so auf den gegenüberliegenden Seiten der transparenten Grundfläche (320) angeordnet sind, dass jeweils zumindest ein kontrastverstärkender Hintergrund (352, 354, 372, 374) aus einer jeweiligen Kameraperspektive hinter dem zu erkennenden Instrument (58, 64, 328) liegt.

11. Vorrichtung nach einem der Ansprüche 1 bis 10, **dadurch gekennzeichnet, dass** die Auflage (124, 174, 206) der Groberkennungseinheit (106, 156, 200, 306) eine Waage (174, 208) aufweist und die Datenverarbeitungsanlage (14, 104, 154, 212, 304) eine zweite Schnittstelle (176, 228) aufweist und ferner so ausgestaltet ist, dass sie über die zweite Schnittstelle (176, 228) Gewichtsdaten des aufgelegten Instrumentensets von der Waage (174, 208) erhält und die erhaltenen Gewichtsdaten in der Datenbank (28, 184, 236) speichern und mittels der Auswerteeinheit (26, 182, 232) mit bereits gespeicherten Gewichtsdaten der optisch identifizierten Instrumente (58, 64, 328) vergleichen kann.

12. Vorrichtung nach einem der Ansprüche 1 bis 11, **dadurch gekennzeichnet, dass** die Groberkennungseinheit (106, 156, 200, 306) eine Kamera (122, 202) aufweist, die so angeordnet und ausgerichtet ist, dass sie Bilddaten eines Instrumentensets auf der Auflage (124, 174, 206) der Groberkennungseinheit (106, 156, 200, 306) aus zumindest einer Perspektive erfassen kann, und die Datenverarbeitungsanlage (14, 104, 154, 212, 304) so ausgestaltet ist, dass sie über eine Schnittstelle (24, 180, 226, 230) Bilddaten von der Kamera (122, 202) der Groberkennungseinheit (106, 156, 200, 306) erhält und die erhaltenen Bilddaten in der Datenbank (28, 184, 236) speichern und mittels der Auswerteeinheit (26, 182, 232) mit bereits gespeicherten Bilddaten von Instrumenten (58, 64, 328) vergleichen und dadurch die jeweiligen Instrumente (58, 64, 328) optisch identifizieren und die Vollständigkeit des Instrumentensets überprüfen kann.

13. Vorrichtung nach Anspruch 12, **dadurch gekennzeichnet, dass** die Kamera (122, 202) der Groberkennungseinheit (106, 156, 200, 306) durch eine weitere Kamera (122, 202) gebildet wird und die Datenverarbeitungsanlage (14, 104, 154, 212, 304) eine dritte Schnittstelle (230) aufweist und so ausgestaltet ist, dass sie über die dritte Schnittstelle (230) Bilddaten von der weiteren Kamera (122, 202) erhält.

14. Vorrichtung nach Anspruch 12 oder 13, **dadurch gekennzeichnet, dass** die Kamera (122, 202) der Groberkennungseinheit (106, 156, 200, 306) in ihrer Position veränderbar angeordnet ist, so dass sie Bilddaten aus zumindest zwei Perspektiven erfassen kann.

15. Vorrichtung nach einem der Ansprüche 1 bis 14, **dadurch gekennzeichnet, dass** die Auflage (124, 174, 206) der Groberkennungseinheit (106, 156, 200, 306) ein Instrumentensieb (134, 172, 210) aufweist, in das die Instrumente (58, 64, 328) gelegt und anschließend als vollständiges Instrumentenset sterilisiert werden können.

16. Verfahren zum Zusammenstellen von medizinischen Instrumentensets mit einer Vorrichtung nach einem der Ansprüche 1 bis 15 das folgende Schritte aufweist:
a) Auflegen von zumindest einem Instrument auf die erste Auflage (16, 108, 158, 312) der Feinerkennungseinheit (12, 102, 152, 302, 351, 371),
b) Erfassen der Bilddaten durch zumindest die erste Kamera (18, 110, 160, 308),
c) Weitergabe der Bilddaten an die Datenverarbeitungsanlage (14, 104, 154, 212,304),
d) Vergleich der Bilddaten mit den in der Datenbank (28, 184, 236) gespeicherten Referenzbildern für die Instrumente (58, 64, 328) des zusammenzustellenden Instrumentensets,
e) Informieren des Benutzers über die Anzeigeeinheit (38, 120, 170, 218) über fehlende oder überflüssige Instrumente beziehungsweise über die Vollständigkeit des Instrumentensets.

17. Verfahren nach Anspruch 16, **dadurch gekennzeichnet, dass** im Schritt b) die Bilddaten durch die erste und zweite Kamera (18, 110, 160, 308; 310) erfasst werden.

18. Verfahren nach Anspruch 16 oder 17, **dadurch gekennzeichnet, dass** zumindest eine Kamera (18, 110, 160, 308; 310; 252) Bilddaten aus zumindest zwei Perspektiven erfasst, indem die jeweilige Kameraposition verändert wird.

19. Verfahren nach einem der Ansprüche 16 bis 18, **dadurch gekennzeichnet, dass** es ferner folgende Schritte aufweist:
f) Ermitteln des Gewichts des Instrumentensets mit der Waage (174, 208),
g) Übermitteln des Gewichts an die Datenverarbeitungsanlage (14, 104, 154, 212,304),
h) Vergleichen des Gewichts mit einem Sollgewicht des Instrumentensets,
i) Informieren des Benutzers über Vollständigkeit oder Fehler des Instrumentensets.

20. Verfahren nach einem der Ansprüche 16 bis 18, **dadurch gekennzeichnet, dass** es ferner folgende Schritte aufweist:
f) Erfassen der Bilddaten des Instrumentensets mit der Kamera (122, 202) der Groberkennungseinheit (106, 156, 200, 306),
g) Übermitteln der Bilddaten an die Datenverarbeitungsanlage (14, 104, 154, 212,304),
h) Vergleichen der Bilddaten mit den in der Datenbank (28, 184, 236) gespeicherten Bilddaten der Instrumente (58, 64, 328), die zu dem Instrumentenset gehören,
l) Informieren des Benutzers über Vollständigkeit oder Fehler des Instrumentensets.

21. Verfahren nach Anspruch 20, **dadurch gekennzeichnet, dass** der Vergleich in Schritt h) anhand von Schlüsselmerkmalen der Instrumente (58, 64, 328) stattfindet.

22. Verfahren nach Anspruch 20 oder 21, **dadurch gekennzeichnet, dass** es zusätzlich zwischen den Schritten h) und l) folgende Schritte aufweist:
i) Ermitteln des Gewichts des Instrumentensets mit der Waage (174, 208),
j) Übermitteln des Gewichts an die Datenverarbeitungsanlage (14, 104, 154, 212,304),
k) Vergleichen des Gewichts mit einem Sollgewicht des Instrumentensets.
